# EUROPEAN PATENT APPLICATION

(11) **EP 2 213 289 A1**
(43) Date of publication of application: **04.08.2010**
(21) Application number: 10005207.5
(22) Date of filing: 04.09.2007
(51) Int. Cl.: A61K 31/473, A61P 3/10

(54) **Combination treatment for diabetes mellitus**

(30) Priority: 07.09.2006 EP 06120305
(62) Divisional of application: 07803217.4
(71) Applicant: Nycomed GmbH, 78467 Konstanz (DE)
(72) Inventor: Klein, Thomas Dr., 78315 Radolfzell (DE); Blaser, Anja Dr., 78465 Konstanz (DE); Rudolph, Bettina Dr., 4052 Basel (CH); Kautz, Ulrich Dr., 78476 Allensbach (DE); Selige, Jens, 78462 Konstanz (DE); Kromer, Wolfgang Prof. Dr., 78464 Konstanz (DE)
(74) Representative: Wild, Robert

(57) **Abstract**

The invention relates to combinations of (2R,4aR,10bR)-6-(2,6-Dimethoxy-pyridin-3-yl)-9-ethoxy-8-methoxy-1,2,3,4,4a,10b-hexahydrophenanthridin-2-ol with other active compounds for the treatment of diabetes mellitus type 2 and/or type 1.

## Description

### Technical field

The invention relates to combinations of a known PDE4 inhibitor with one or two other active compounds which are used in the treatment of diabetes mellitus type 2 and/or diabetes mellitus type 1; the invention also relates to pharmaceutical compositions, combination products and kits containing these combinations as well as uses of such combinations in the treatment of diabetes mellitus type 2 and/or diabetes mellitus type 1.

### Background of the invention

Diabetes mellitus is on the rise worldwide and is considered to be at an epidemic level by the World Health Organization. The clinical manifestation and progression of diabetes often vary considerably between countries and commonly between ethnic groups in the same country. Currently diabetes affects 151 million people worldwide and an estimate 300 million people in 2025. There are two main forms of diabetes. Type 1 (insulin-dependent diabetes mellitus, IDDM) is due primarily to autoimmune-mediated destruction of pancreatic β-cells, resulting in absolute insulin deficiency. It is the second most common chronic disease of children. By contrast, type 2 diabetes (non-insulin-dependent diabetes mellitus, NIDDM) is characterized by insulin-resistance and inadequate insulin secretion. A significant fraction of individuals originally diagnosed with type 2 diabetes evolve with time to a type 1 state, which is defined as exhibiting anti-β-cell autoimmunity.

Because genetic factors contribute to the development of diabetes, the disease displays a strong familial aggregation. Although there are monogenic syndromes of insulin resistance, in which a definite gene has been identified as the cause of insulin resistance, these are relative rare. The more common presentation of diabetes appears to be polygenic. Additionally, behavioural- and lifestyle-related risk factors exist. Type 2 diabetes is increasingly common primarily because of increases in the prevalence of a sedentary lifestyle and obesity. One of the major arguments for the role of behavioural factors in the etiology of diabetes has been the rapid increase in the prevalence and incidence of the disease in populations undergoing rapid westernization. The westernization transition is usually accompanied by increases in obesity, decreases in physical activity and alterations in dietary intake toward more calories, fat and non-complex carbohydrates.

Plasma glucose concentrations are normally maintained within a fairly narrow range despite wide fluctuations in the body's supply (e.g. meals) and demand (e.g. exercise) for nutrients. After an overnight fast, insulin-independent tissues, the brain (50%) and splanchnic organs (25%), account for most of the total body glucose disposal. Insulin-dependent tissues, adipose tissue and primarily skeletal muscles, are responsible for the remaining 25% of glucose utilization. This basal glucose uptake is precisely matched by the release of glucose from the liver. In response to hyperglycemia after a meal, pancreatic insulin secretion is stimulated and the combination of hyperinsulinemia plus hyperglycemia promotes glucose uptake (by splanchnic and peripheral, primarily muscle, tissues) and suppresses hepatic glucose production. It follows, therefore, that defects at the level of the β-cell, muscle and liver can lead to the development of glucose intolerance and diabetes mellitus. All the abnormalities in diabetes basically result from an imbalance between insulin sensitivity and insulin secretion. The initial stage of diabetes is characterised by impaired glucose tolerance and postprandial hyperglycemia. As the disease progresses, fasting hyperglycemia is observed.

The earliest detectable abnormality in NIDDM is an impairment in the body's ability to respond to insulin. Because the pancreas is able to appropriately augment its secretion of insulin to offset the insulin resistance, glucose tolerance remains normal. With time, however, the beta-cell fails to maintain its high rate of insulin secretion and the insulin resistance leads to the development of impaired glucose tolerance and eventually overt diabetes mellitus. The cause of pancreatic "exhaustion" remains unknown, however lipo- and glucotoxicity with an increased level of oxygen radical stress are discussed more recently. Insulin resistance in NIDDM involves both hepatic and peripheral tissues. In response to both endogenously secreted or exogenously administered insulin, hepatic glucose production fails to suppress normally and muscle glucose uptake is diminished. The accelerated rate of hepatic glucose output is due mainly to augmented gluconeogenesis. In muscle many cellular defects in insulin action have been described including impaired insulin-receptor tyrosine kinase activity, diminished glucose transport, and reduced glycogen synthase and pyruvate dehydrogenase activities. The abnormalities account for disturbances in the two major intracellular pathways of glucose disposal, glycogen synthesis and glucose oxidation. In the earliest stages of NIDDM, the major defect involves the inability of insulin to promote glucose uptake and storage as glycogen. Other potential mechanisms that have been put forward to explain the glucose intolerance include increased levels of free fatty acids, chronic low-grade activation of the immune system (increased levels of TNFα and IL6) , altered skeletal muscle blood flow, increased conversion of amylin to its insoluble amyloid form and glucose toxicity.

Diabetes is associated with a variety of physiologic disorders such as hypertension and dyslipidemia. Diabetes also increases the risk of macrovascular (coronary artery disease, stroke, amputation) and microvascular (blindness, renal failure, neuropathies) diseases. Myocardial infarction, stroke or renal failure are the cause of death for more than 70% of diabetes patients. The huge mortality and debilitating neuropathies associated with diabetes underline the importance of active medical intervention.

There are several ways to counteract diabetes. The first is lifestyle adjustments aimed at improving endogenous insulin sensitivity. This can be achieved by increased physical activity and bodyweight reduction with diet and behavioural modification. Unfortunately, most people with non-insulin-dependent diabetes mellitus never receive sufficient nutritional education or are not capable of complying with a strict diet regimen.

Another therapeutic way involves increasing insulin availability by the administration of exogenous insulin, insulin analogues and insulin secretagogues such as sulphonylureas. The primary mode of action of sulphonylureas is through the depolarisation of the pancreatic β-cells by blocking the ATP-dependent potassium channels and causing an influx of calcium ions, which stimulate insulin secretion.

Biguanides, of which metformin is the most commonly used, also have proven to be effective anti-hyperglycemic agents. Metformin reduces hepatic gluconeogenesis and basal hepatic glucose output. Oral antidiabetics such as sulphonylureas and metformin as monotherapy or in combination have been shown to decrease fasting plasma glucose levels, but postprandial hyperglycemia persists in more than 60% of patients and probably accounts for sustained increases of hemoglobin A_{1C} levels.

α-Glucosidase inhibitors, e.g. acarbose and miglitol, primarily target postprandial hyperglycemia. The therapy of diabetes mellitus with α-glucosidase inhibitors is based on a delayed intestinal degradation of starch and sucrose. These carbohydrates must be hydrolysed by α-glucosidases to monosaccharides before they can be transported through the mucosa of the small intestine. The reversible inhibition of the brush border glucosidases results in redistribution of carbohydrate absorption from the upper portion of the gut to a more extended surface area covering the whole length of the small intestine. This is accompanied by a delayed absorption of monosaccharides and a decrease in the postprandial elevation of blood glucose.

Another class of antidiabetic drugs are thiazolidinediones, such as rosiglitazone and pioglitazone, which are insulin sensitizers and act through activation of peroxisome proliferator-activated receptor γ (PPARγ). PPARγ is mainly expressed in adipose tissues, plays an important role in adipogenesis and modifies fatty acid synthesis and storage. Binding of rosiglitazone to PPARγ results in reduced endogenous glucose production and increased blood glucose uptake. It increases the sensitivity of skeletal muscle, liver and adipose tissues to insulin. Improvements in glucose metabolism with rosiglitazone treatment are closely correlated with decreased plasma free fatty acid metabolism. The stimulation by rosiglitazone of PPARγ in adipose tissue and subsequent adipocyte differentiation results in the generation of more, but smaller, adipocytes which are more insulin sensitive and produce less free fatty acid, TNFα and leptin.

In the International Patent Application WO9914239 compositions for treating diabetes mellitus and obesity are disclosed. The compositions contain at least two of the active agents A, B and C, wherein A is at least one hormone which stimulates the production of cAMP, B is at least one substance which inhibits the breakdown of a cyclic nucleotide, and C is at least one hormone which stimulates the production of cGMP. In the International Patent Application WO0135979 the combined use of a PDE3 and a PDE4 inhibitor for the treatment of obesity is disclosed. In the International Patent Application WO0213798 the use of a selective cGMP PDE5 inhibitor for the treatment of Insulin Resistance Syndrome is disclosed, wherein the Insulin Resistance Syndrome is defined as the concomitant existence of two or more disease states selected from dyslipidemia, hypertension, type 2 diabetes mellitus, impaired glucose tolerance, a family history of diabetes, hyperuricaemia and/or gout, a pro-coalgulant state, atherosclerosis and truncal obesity. In the unexamined German application DE 10150517 tetra-hydropyridazin-3-one derivatives are described which may be useful inter alia for the treatment of diabetes mellitus. In the International Patent Application WO2005023253 several PDE4 inhibitors are disclosed to be useful in the treatment of diabetes mellitus. In Diabetes 47, pp. 570-575, 1998 is disclosed that pentoxyfylline and rolipram may be effective in the treatment of autoimmune diabetes or other conditions characterized by excessive production of inflammatory cytokines.

It is an object of the present invention to provide combinations and methods of treatment that take advantage of the different therapeutic pathways of a PDE4 inhibitor on the one hand side and other known anti-diabetic compounds on the other hand side to more effectively treat diabetes mellitus type 2 and/or type 1.

### Description of the invention

The invention is based on the expectation that the use of the selective PDE4 inhibitor (2R,4aR,10bR)-6-(2,6-Dimethoxy-pyridin-3-yl)-9-ethoxy-8-methoxy-1,2,3,4,4a,10b-hexahydrophenanthridin-2-ol (hereinafter referred to as "Compound A") or a pharmaceutical acceptable salt thereof in combination with one or two other active compound(s) or pharmaceutically acceptable salt(s) thereof which is (are) used in the treatment of diabetes mellitus type 2 and/or type 1 leads to beneficial effects in the treatment of diabetes mellitus type 2 and/or type 1 in comparison to the treatment with either the selective PDE4 inhibitor (2R,4aR,10bR)-6-(2,6-Dimethoxy-pyridin-3-yl)-9-ethoxy-8-methoxy-1,2,3,4,4a,10b-hexahydrophenanthridin-2-ol or the above-mentioned active compound(s) alone.

Therefore, according to a first aspect of the present invention there is provided a pharmaceutical composition comprising a pharmaceutical formulation including Compound A or a pharmaceutically acceptable salt thereof, one other active compound or a pharmaceutically acceptable salt thereof which is used in the treatment of diabetes mellitus type 2 and/or type 1, and at least one pharmaceutically acceptable auxiliary.

As an embodiment of the first aspect of the present invention there is provided a pharmaceutical composition comprising a pharmaceutical formulation including an amount of Compound A or a pharmaceutically acceptable salt thereof, an amount of one other active compound or a pharmaceutically acceptable salt thereof which is used in the treatment of diabetes mellitus type 2 and/or type 1, wherein the first amount and the second amount together comprise an effective amount for the treatment of diabetes mellitus type 2 and/or type 1, and at least one pharmaceutically acceptable auxiliary. According to a second aspect of the present invention there is provided a pharmaceutical composition comprising a pharmaceutical formulation including Compound A or a pharmaceutical acceptable salt thereof, two other active compounds or pharmaceutically acceptable salts thereof which are used in the treatment of diabetes mellitus type 2 and/or type 1, and at least one pharmaceutically acceptable auxiliary.

As an embodiment of the second aspect of the present invention there is provided a pharmaceutical composition comprising a pharmaceutical formulation including an amount of Compound A or a pharmaceutically acceptable salt thereof, amounts of two other active compounds or pharmaceutically acceptable salts thereof which are used in the treatment of diabetes mellitus type 2 and/or type 1, wherein the first, second and third amount together comprise an effective amount for the treatment of diabetes mellitus type 2 and/or type 1, and at least one pharmaceutically acceptable auxiliary.

The above-mentioned pharmaceutical compositions provide for the administration of Compound A or a pharmaceutically acceptable salt thereof with one or two other active compound(s) or pharmaceutically acceptable salts thereof which is (are) used in the treatment of diabetes mellitus type 2 and/or type 1 and is thus presented as a single formulation.

Alternatively, Compound A or a pharmaceutically acceptable salt thereof and the one or two other active compound(s) or pharmaceutically acceptable salt(s) thereof which is (are) used in the treatment of diabetes mellitus type 2 and/or type 1 may be presented as separate formulations, wherein at least one of those formulations comprises Compound A or a pharmaceutically acceptable salt thereof and at least one comprises one or two other active compound(s) or pharmaceutically acceptable salt(s) thereof which is (are) used in the treatment of diabetes mellitus type 2 and/or type 1.

Thus, there is further provided:
A combination product comprising the components: (A) Compound A or a pharmaceutically acceptable salt thereof; and (B) one other active compound or pharmaceutically acceptable salt thereof which is used in the treatment of diabetes mellitus type 2 and/or type 1, wherein each of the components (A) and (B) is formulated in admixture with at least one pharmaceutically acceptable auxiliary.

As an embodiment of the above-mentioned combination product there is provided a combination product comprising the components: (A) an amount of Compound A or a pharmaceutically acceptable salt thereof; and (B) an amount of one other active compound or pharmaceutically acceptable salt thereof which is used in the treatment of diabetes mellitus type 2 and/or type 1, wherein the first and the second amount together comprise an effective amount for the treatment of diabetes mellitus type 2 and/or type 1 and wherein each of the components (A) and (B) is formulated in admixture with at least one pharmaceutically acceptable auxiliary.

A combination product comprising the components: (A) Compound A or a pharmaceutically acceptable salt thereof; (B) one other active compound or pharmaceutically acceptable salt thereof which is used in the treatment of diabetes mellitus type 2 and/or type 1; and (C) still another active compound or pharmaceutically acceptable salt thereof which is used in the treatment of diabetes mellitus type 2 and/or type 1, wherein each of the components (A), (B) and (C) is formulated in admixture with at least one pharmaceutically acceptable auxiliary.

As an embodiment of the above-mentioned combination product there is provided a combination product comprising the components: (A) an amount of Compound A or a pharmaceutically acceptable salt thereof; (B) an amount of one other active compound or pharmaceutically acceptable salt thereof which is used in the treatment of diabetes mellitus type 2 and/or type 1; and (C) an amount of still another active compound or pharmaceutically acceptable salt thereof which is used in the treatment of diabetes mellitus type 2 and/or type 1, wherein the first, second and third amount together comprise an effective amount for the treatment of diabetes mellitus type 2 and/or type 1 and wherein each of the components (A), (B) and (C) is formulated in admixture with at least one pharmaceutically acceptable auxiliary.

A kit comprising the components: (A) a pharmaceutical formulation including Compound A or a pharmaceutically acceptable salt thereof, in admixture with at least one pharmaceutically acceptable auxiliary; and (B) a pharmaceutical formulation including one other active compound or pharmaceutically acceptable salt thereof which is used in the treatment of diabetes mellitus type 2 and/or type 1, in admixture with at least one pharmaceutically acceptable auxiliary.

As an embodiment of the above-mentioned kit there is provided a kit comprising the components: (A) a pharmaceutical formulation including an amount of Compound A or a pharmaceutically acceptable salt thereof, in admixture with at least one pharmaceutically acceptable auxiliary; and (B) a pharmaceutical formulation including an amount of one other active compound or pharmaceutically acceptable salt thereof which is used in the treatment of diabetes mellitus type 2 and/or type 1, in admixture with at least one pharmaceutically acceptable auxiliary, wherein the first and the second amount together comprise an effective amount for the treatment of diabetes mellitus type 2 and/or type 1.

A kit comprising the components: (A) a pharmaceutical formulation including Compound A or a pharmaceutically acceptable salt thereof, in admixture with at least one pharmaceutically acceptable auxiliary; (B) a pharmaceutical formulation including an amount of one other active compound or pharmaceutically acceptable salt thereof which is used in the treatment of diabetes mellitus type 2 and/or type 1, in admixture with at least one pharmaceutically acceptable auxiliary; and (C) a pharmaceutical formulation including still another active compound or a pharmaceutically acceptable salt thereof which is used in the treatment of diabetes mellitus type 2 and/or type 1, in admixture with at least one pharmaceutically acceptable auxiliary.

As an embodiment to the above-mentioned kit there is provided a kit comprising the components: (A) a pharmaceutical formulation including an amount of Compound A or a pharmaceutically acceptable salt thereof, in admixture with at least one pharmaceutically acceptable auxiliary; (B) a pharmaceutical formulation including an amount of one other active compound or pharmaceutically acceptable salt thereof which is used in the treatment of diabetes mellitus type 2 and/or type 1, in admixture with at least one pharmaceutically acceptable auxiliary; and (C) a pharmaceutical formulation including an amount of still another active compound or a pharmaceutically acceptable salt thereof which is used in the treatment of diabetes mellitus type 2 and/or type 1, in admixture with at least one pharmaceutically acceptable auxiliary, wherein the first, second and third amount together comprise an effective amount for the treatment of diabetes mellitus type 2 and/or type 1.

In addition, to the components A and B (or A, B and C) the kits according to the invention may include information for the sequential or separate administration of the components to the patient in need of diabetes mellitus type 2 and/or type 1 therapy.

The combinations according to the present invention are used for the treatment of diabetes mellitus type 2 and/or type 1, preferably for the treatment of diabetes mellitus type 2.

Therefore, further aspects of the invention are:
The use of Compound A or a pharmaceutically acceptable salt thereof and one other active compound or pharmaceutically acceptable salt thereof which is used in the treatment of diabetes mellitus type 2 and/or type 1 for the manufacture of a medicament, in particular a pharmaceutical composition according to the invention, for the treatment of diabetes mellitus type 2 and/or type 1.

The use of Compound A or a pharmaceutically acceptable salt thereof and two other active compounds or pharmaceutically acceptable salts thereof which are used in the treatment of diabetes mellitus type 2 and/or type 1 for the manufacture of a medicament, in particular a pharmaceutical composition according to the invention, for the treatment of diabetes mellitus type 2 and/or type 1.

The use of Compound A or a pharmaceutically acceptable salt thereof and one other active compound or pharmaceutically acceptable salt thereof which is used in the treatment of diabetes mellitus type 2 and/or type 1 for the manufacture of a sequentially or separately co-administrable medicament, in particular the combination product or kit according to the invention, for the treatment of diabetes mellitus type 2 and/or type 1.

The use of Compound A or a pharmaceutically acceptable salt thereof and two other active compounds or pharmaceutically acceptable salts thereof which are used in the treatment of diabetes mellitus type 2 and/or type 1 for the manufacture of a sequentially or separately co-administrable medicament, in particular a combination product or kit according to the invention, for the treatment of diabetes mellitus type 2 and/or type 1.

Compound A or a pharmaceutically acceptable salt thereof and one other active compound or pharmaceutically acceptable salt thereof which is used in the treatment of diabetes mellitus type 2 and/or type 1 for the treatment of diabetes mellitus type 2 and/or type 1.

Compound A or a pharmaceutically acceptable salt thereof and two other active compounds or pharmaceutically acceptable salts thereof which are used in the treatment of diabetes mellitus type 2 and/or type 1 for the treatment of diabetes mellitus type 2 and/or type 1.

A pharmaceutical composition, combination product or kit according to the invention for the treatment of diabetes mellitus type 2 and/or type 1.

Another aspect of the present invention is a method for treating diabetes mellitus type 2 and/or type 1 comprising administering to a patient in need thereof a pharmaceutical composition comprising a pharmaceutical formulation including an amount of Compound A or a pharmaceutically acceptable salt thereof, an amount of one other active compound or a pharmaceutically acceptable salt thereof which is used in the treatment of diabetes mellitus type 2 and/or type 1, wherein the first amount and the second amount together comprise an effective amount for the treatment of diabetes mellitus type 2 and/or type 1, and at least one pharmaceutically acceptable auxiliary.

Still another aspect of the present invention is a method for treating diabetes mellitus type 2 and/or type 1 comprising administering to a patient in need thereof a pharmaceutical composition comprising a pharmaceutical formulation including an amount of Compound A or a pharmaceutically acceptable salt thereof, amounts of two other active compounds or pharmaceutically acceptable salts thereof which are used in the treatment of diabetes mellitus type 2 and/or type 1, wherein the first, second and third amount together comprise an effective amount for the treatment of diabetes mellitus type 2 and/or type 1, and at least one pharmaceutically acceptable auxiliary.

A further aspect of the present invention is a method for treating diabetes mellitus type 2 and/or type 1 comprising administering to a patient in need thereof a combination product comprising the components:
(A) an amount of Compound A or a pharmaceutically acceptable salt thereof; and
(B) an amount of one other active compound or a pharmaceutically acceptable salt thereof which is used in the treatment of diabetes mellitus type 2 and/or type 1,
wherein the first and second amount together comprise an effective amount for the treatment of diabetes mellitus type 2 and/or type 1;
wherein each of the components (A) and (B) is formulated in admixture with at least one pharmaceutically acceptable auxiliary;
and wherein the components (A) and (B) are administered sequentially or separately.

Still a further aspect of the present invention is a method for treating diabetes mellitus type 2 and/or type 1 comprising administering to a patient in need thereof a combination product comprising the components:
(A) an amount of Compound A or a pharmaceutically acceptable salt thereof;
(B) an amount of one other active compound or a pharmaceutically acceptable salt thereof which is used in the treatment of diabetes mellitus type 2 and/or type 1; and
(C) an amount of still another active compound or a pharmaceutically acceptable salt thereof which is used in the treatment of diabetes mellitus type 2 and/or type 1,
wherein the first, second and third amount together comprise an effective amount for the treatment of diabetes mellitus type 2 and/or type 1;
wherein each of the components (A), (B) and (C) is formulated in admixture with at least one pharmaceutically acceptable auxiliary;
and wherein the components (A), (B) and (C) are administered sequentially or separately.

The pharmaceutical compositions according to the invention may be prepared by mixing the first active agent with the second (and optionally third) active agent.

In the above-mentioned mixing process the first active agent and the second (and optionally third) active agent can
a) in a first step be mixed as such, afterwards be processed with at least one pharmaceutically acceptable auxiliary and finally, for example, be pressed to tablets or caplets
   or
b) in a first step separately be processed with at least one pharmaceutically acceptable auxiliary to give granules or pellets containing each only one of the two (or three) active agents; the pellets or granules for their part then can be mixed in an appropriate ratio and either pressed - optionally with further pharmaceutically acceptable auxiliaries - to give, for example tablets or caplets, or can be filled in loose form in capsules.

Therefore, in a still further aspect of the present invention there is provided a process for the preparation of a pharmaceutical composition which comprises mixing a first active agent, which is Compound A or a pharmaceutically acceptable salt thereof with a second (and optionally a third) active agent, which is (are) one or two other active compounds which is (are) used in the treatment of diabetes mellitus type 2 and/or type 1.

Simultaneous administration of Compound A or a pharmaceutically acceptable salt thereof and one or two other active compound(s) or pharmaceutically acceptable salt(s) thereof which is (are) used in the treatment of diabetes mellitus type 2 and/or type 1 can be preferably accomplished, by administering to the patient in need of diabetes mellitus type 2 and/or type 1 therapy the pharmaceutical composition according to the invention in one dosage form, such as for example, in a single capsule, tablet or injection.

Components (A), (B) and optionally (C) of the combination product as well as of the kit may be administered sequentially or separately over the course of the treatment of diabetes mellitus type 2 and/or type 1.

Sequential or separate administration of Compound A or a pharmaceutically acceptable salt thereof and one or two other active compound(s) or pharmaceutically acceptable salt(s) thereof which is (are) used in the treatment of diabetes mellitus type 2 and/or type 1 can be preferably accomplished, by administering to the patient in need of diabetes mellitus type 2 and/or type 1 therapy components (A), (B) and optionally (C) of the combination product or the kit according to the invention in (multiple) separate dosage forms, such as for example, in separate capsules, tablets or injections.

In an alternative, one (or two) of the components (A), (B) and optionally (C) may be formulated as tablet or capsule and the remaining component(s) may be formulated for administration, for example, by injection or inhalation.

Sequential administration encompasses a short time period between the administration of components (A), (B) and optionally (C) of the combination product or the kit according to the invention (for example, the time that is needed to swallow one tablet after the other).

Separate administration encompasses both short and long time periods between the administration of components (A), (B) and optionally (C) of the combination product or the kit according to the invention. However, for the purposes of the present invention at least one of the components is administered while the other component(s) is (are) still having an effect on the patient being treated. In a preferred embodiment of the invention the effect on the patient being treated is a synergistic effect.

The combined administration of Compound A or a pharmaceutically acceptable salt thereof and one or two other active compound(s) or pharmaceutically acceptable salt(s) thereof which is (are) used in the treatment of diabetes mellitus type 2 and/or type 1, either in form of the pharmaceutical composition, combination product or kit according to the invention, lead to an effective treatment of diabetes type 2 and/or type 1, and in a preferred embodiment is superior to the use of either active agent alone. Moreover, in a particularly preferred embodiment, the combined administration of Compound A or a pharmaceutically acceptable thereof and one or two other active compound(s) or pharmaceutically acceptable salt(s) thereof which is (are) used in the treatment of diabetes type 2 and/or type 1 shows a synergistic efficacy for treating diabetes mellitus type 2 and/or type 1.

As used herein, the term "synergistic" refers to the combination of Compound A or a pharmaceutically acceptable salt thereof with one or two other active compound(s) or pharmaceutically acceptable salt(s) thereof which is (are) used in the treatment of diabetes mellitus type 2 and/or type 1 either in form of the pharmaceutical composition, combination product or kit according to the invention having an efficacy for the treatment of diabetes mellitus type 2 and/or type 1 that is greater than would be expected from the sum of their individuals effects. The synergistic effects of the embodiments of the present invention encompass additional unexpected advantages for the treatment of diabetes mellitus type 2 and/or type 1. Such additional advantages may include, but are not limited to, lowering the required dose of one or more of the active agents of the combination, reducing the side effects of one or more of the active agents of the combination or rendering one or more of the active agents more tolerable to the patient in need of a diabetes type 2 and/or type 1 therapy. The combined administration of Compound A or a pharmaceutically acceptable salt thereof and one or two other active compound(s) or pharmaceutically acceptable salts thereof which is (are) used in the treatment of diabetes mellitus type 2 and/or type 1 may also be useful for decreasing the required number of separate dosages, thus, potentially improving compliance of the patient in need of diabetes mellitus type 2 and/or type 1 therapy.

The term "active agent" as used herein refers either to Compound A or a pharmaceutically acceptable salt thereof or to the active compound(s) or pharmaceutically acceptable salt(s) thereof, which is (are) used in the treatment of diabetes mellitus type 2 and/or type 1.

The term "active compound" as used herein refers to a compound useful in the treatment of a disease, here in particular in the treatment of diabetes mellitus type 2 and/or type 1.

The term "effective amount" as used herein refers to a therapeutically effective amount for treating diabetes mellitus type 2 and/or type 1. In case of a combination therapy the term "effective amount" refers to the sum of the amounts of the combination partners, which is therapeutically effective for the treatment of diabetes mellitus type 2 and/or type 1.

In case of the pharmaceutical compositions, combination products and kits according to the invention the term "amount" is mentioned in connection with the pharmaceutical formulations which form part of the pharmaceutical compositions, or in connection with the components which form part of the combination products or kits. The term "effective amount" is mentioned in phrases, such as "wherein the first and second amount (or the first, second and third amount) together comprise an effective amount for the treatment of diabetes mellitus type 2 and/or type 1". In case, a once daily administration of the pharmaceutical formulation of the pharmaceutical composition, or a once daily administration of the components of the combination product is intended, the respective amount(s) correspond to the daily dosage necessary for the treatment of diabetes mellitus type 2 and/or type 1. In case, a twice a day administration of the pharmaceutical formulation of the pharmaceutical composition, or a twice a day administration of the components (or one of the components) of the combination product or kit is intended, the respective amount(s) correspond to the daily dosage necessary for the treatment of diabetes mellitus type 2 and/or type 1 multiplied by the factor 0.5. In case, a three times a day administration of the pharmaceutical formulation of the pharmaceutical composition, or a three times a day administration of the components (or one of the components) of the combination product or kit is intended, the respective amount(s) correspond to the daily dosage necessary for the treatment of diabetes mellitus type 2 and/or type 1 multiplied by the factor 0.33 and so on.

The term "patient" includes both humans and other mammals. In a preferred embodiment of the invention the term "patient" stands for humans.

The term "one other active compound" in connection with the term "still another active compound" means that the two mentioned active compounds are not identical. As well, the term "two other active compounds" stands for two active compounds that are not identical.

The term "Compound A" as used herein stands for (2R,4aR,10bR)-6-(2,6-Dimethoxy-pyridin-3-yl)-9-ethoxy-8-methoxy-1,2,3,4,4a,10b-hexahydrophenanthridin-2-ol.

In one embodiment of the present invention a preferred pharmaceutically acceptable salt of Compound A is selected from the tosylate, esylate, hydrobromide and hydrochloride salt of Compound A.

In another embodiment of the present invention a preferred pharmaceutically acceptable salt of Compound A is the hydrochloride salt of Compound A.

Additional information with regard to the preparation and suitable dosage forms of Compound A and the pharmaceutically acceptable salts thereof can be found in the following patents/patent applications: WO2005/085225 and PCT/EP2006/060377 (WO2006092422).

Non-limiting examples of other active compounds which are used in the treatment of diabetes mellitus type 2 and/or type 1 are provided in the following list:
- Insulin and insulin analogues
- Glucagon-Like-Peptide-1 (GLP-1) receptor agonists
- Sulfonylurea agents
- Biguanide agents
- Alpha-glucosidase inhibitors
- PPAR-Agonists
- Meglitinide agents
- Dipeptidyl-peptidase (DPP) IV inhibitors
- PDE1, PDE5, PDE9, PDE10 or PDE11 inhibitors
- Amylin agonists
- Cinnamon
- Glucagon receptor antagonists
- Glycogen-Phosphorylase inhibitors
- Fructose-1,6-Bisphosphate inhibitors
- Cannabinoid (CB1) receptor antagonists
- Anti-obesity drugs such as appetite suppressors, satiety increasing substances, and energy expenditure increasing drugs
and pharmaceutically acceptable salts thereof.

In one embodiment of the invention, the one ot two other active compound(s) which is (are) used in the treatment of diabetes mellitus type 2 and/or type 1 is (are) selected from the group consisting of
- Insulin and insulin analogues
- Glucagon-Like-Peptide-1 (GLP-1) receptor agonists
- Sulfonylurea agents
- Biguanide agents
- Alpha-glucosidase inhibitors
- PPAR-Agonists
- Meglitinide agents
- Dipeptidyl-peptidase (DPP) IV inhibitors
- PDE1, PDE5, PDE9, PDE10 or PDE11 inhibitors
- Amylin agonists
- Cinnamon
- Glucagon receptor antagonists
- Glycogen-Phosphorylase inhibitors
- Fructose-1,6-Bisphosphate inhibitors
- Cannabinoid (CB1) receptor antagonists
- Anti-obesity drugs such as appetite suppressors, satiety increasing substances, and energy expenditure increasing drugs
and pharmaceutically acceptable salts thereof.

In another embodiment of the present invention the other active compound which is used in the treatment of diabetes mellitus type 2 and/or type 1 is insulin. Specific examples of insulin include, but are not limited to Humulin® [human insulin, (rDNA origin)], Novolin® [human insulin, (rDNA origin)], Velosulin® BR [human buffered regular insulin, (rDNA origin)] and Exubera® [human insulin, inhaled].

In another embodiment of the present invention the other active compound which is used in the treatment of diabetes mellitus type 2 and/or type 1 is an insulin analogue or a pharmaceutically acceptable salt thereof. Specific examples of insulin analogues include, but are not limited to, novarapid, insulin detemir, insulin lispro, insulin glargine, insulin zinc suspension and Lys-Pro insulin.

In another embodiment of the present invention the other active compound which is used in the treatment of diabetes mellitus type 2 and/or type 1 is a Glucagon-Like-Peptide-1 receptor agonist or a pharmaceutically acceptable salt thereof. Specific examples of Glucagon-Like-Peptide-1 receptor agonists include, but are not limited to BIM-51077 (CAS-No. 275371-94-3), EXENATIDE (CAS-No. 141758-74-9), LIRAGLUTIDE (CAS-No. 20656-20-2), ALBIGLUTIDE (CAS-No. 782500-75-8) and ZP-10 (CAS-No. 320367-13-3). A preferred Glucagon-Like-Peptide-1 receptor agonist is EXENATIDE.

In another embodiment of the present invention the other active compound which is used in the treatment of diabetes mellitus type 2 and/or type 1 is a sulfonylurea agent or a pharmaceutically acceptable salt thereof. Specific examples of sulfonylurea agents include, but are not limited to, TOLBUTAMIDE (CAS-No. 000064-77-7), TOLAZAMIDE (CAS-No. 001156-19-0), GLIPIZIDE (CAS-No. 029094-61-9), CARBUTAMIDE (CAS-No. 000339-43-5), GLISOXEPIDE (CAS-No. 025046-79-1), GLISENTIDE (CAS-No. 032797-92-5), GLIBORNURIDE (CAS-No. 026944-48-9), GLIBENCLAMIDE (CAS-NO. 010238-21-8), GLIQUIDONE (CAS-No. 033342-05-1), GLIMEPIRIDE (CAS-No. 093479-97-1) and GLICLAZIDE (CAS-No. 021187-98-4).

In another embodiment of the present invention the pharmaceutically acceptable salt of TOLBUTAMIDE is the sodium salt of TOLBUTAMIDE. In another embodiment of the present invention the pharmaceutically acceptable salt of GLIQUIDONE is the sodium salt of GLIQUIDONE.

In another embodiment of the present invention the other active compound which is used in the treatment of diabetes mellitus type 2 and/or type 1 is a biguanide agent or a pharmaceutically acceptable salt thereof. A specific example of a biguanide agent includes, but is not limited to METFORMIN (CAS-No. 000657-24-9).

In another embodiment of the present invention the pharmaceutically acceptable salt of METFORMIN is the hydrochloride salt of METFORMIN.

In another embodiment of the present invention the other active compound which is used in the treatment of diabetes mellitus type 2 and/or type 1 is an alpha-glucosidase-inhibitor or a pharmaceutically acceptable salt thereof. Specific examples of alpha-glucosidase-inhibitors include, but are not limited to ACARBOSE (Cas-No. 056180-94-0), MIGLITOL (CAS-No. 072432-03-2) and VOGLIBOSE (CAS-No. 083480-29-9).

In another embodiment of the present invention the other active compound which is used in the treatment of diabetes mellitus type 2 and/or type 1 is a PPAR-agonist or a pharmaceutically acceptable salt thereof. Specific examples of PPAR-agonists include, but are not limited to MURAGLITAZAR (CAS-No. 331741-94-7), ROSIGLITAZONE (CAS-NO. 122320-73-4), PIOGLITAZONE (CAS-No.111025-46-8), FARGLITAZAR (CAS-No. 196808-45-4), NAVEGLITAZAR (CAS-No. 476436-68-7), NETOGLITAZONE (CAS-NO. 161600-01-7), RIVOGLITAZONE (CAS-No. 185428-18-6), K-111 (CAS-No. 221564-97-2), SODELGLITAZAR (= GW-677954; CAS-No. 622402-24-8) and (-)-Halofenate (CAS-No. 024136-23-0). Preferred PPAR-agonists are ROSGLITAZONE and PIOGLITAZONE.

In another embodiment of the present invention the pharmaceutically acceptable salt of ROSIGLITAZONE is the maleate salt of ROSIGLITAZONE. In another embodiment of the present invention the pharmaceutically acceptable salt of RIVOGLITAZONE is the hydrochloride salt of RIVOGLITAZONE. In another embodiment of the present invention the pharmaceutically acceptable salt of K-111 is the sodium salt of K-111. In another embodiment of the present invention the pharmaceutically acceptable salt of PIOGLITAZONE is the dihydrochloride salt of PIOGLITAZONE.

In another embodiment of the present invention the other active compound which is used in the treatment of diabetes mellitus type 2 and/or type 1 is a meglitinide agent or a pharmaceutically acceptable salt thereof. Specific examples of meglitinide agents include, but are not limited to REPAGLINIDE (CAS-No. 135062-02-1), NATEGLINIDE (CAS-No. 105816-04-4) and MITIGLINIDE (CAS-No. 145375-43-5).

In another embodiment of the present invention the pharmaceutically acceptable salts of MITIGLINIDE are the monopotassium or the calcium salt of MITIGLINIDE.

In another embodiment of the present invention the other active compound which is used in the treatment of diabetes mellitus type 2 and/or type 1 is a DPP-IV inhibitor or a pharmaceutically acceptable salt thereof. Specific examples of DPP IV inhibitors include, but are not limited to SITAGLIPTIN (CAS-No. 486460-32-6), SAXAGLIPTIN (CAS-No. 361442-04-8), VILDAGLIPTIN (CAS-No. 274901-16-5), DENAGLIPTIN (CAS-No. 483369-58-0), ALOGLIPTIN (CAS-No. 850649-61-5) and P32/98 (CAS-No. 251572-70-0).

In another embodiment of the present invention the pharmaceutically acceptable salt of SITAGLIPTIN is the phosphate salt of SITAGLIPTIN. In another embodiment of the present invention the pharmaceutically acceptable salt of ALOGLIPTIN is the benzoate salt of ALOGLIPTIN. In another embodiment of the present invention the pharmaceutically acceptable salts of P32/98 are the fumarate or hydrochloride salt of P32/98.

In another embodiment of the present invention the other active compound which is used in the treatment of diabetes mellitus type 2 and/or type 1 is a PDE5 inhibitor or a pharmaceutically acceptable salt thereof. Specific examples of PDE5 inhibitors include, but are not limited to SILDENAFIL (CAS-No. 139755-83-2), VARDENAFIL (CAS-No. 224785-90-4), TADALAFIL (CAS-No. 171596-29-5), UDENAFIL (CAS-No. 268203-93-6) and AVANAFIL (CAS-No. 330784-47-9).

In another embodiment of the present invention the pharmaceutically acceptable salts of SILDENAFIL are the hemi-citrate, the citrate or the mesilate salt of SILDENAFIL; particularly preferred is the citrate salt of SILDENAFIL. In another embodiment of the present invention the pharmaceutically acceptable salts of VARDENAFIL are the mono-hydrochloride salt of VARDENAFIL or the dihydrochloride salt of VARDENAFIL. In another embodiment of the present invention the pharmaceutically acceptable salt of AVANAFIL is the besilate salt of AVANAFIL.

In another embodiment of the present invention the other active compound which is used in the treatment of diabetes mellitus type 2 and/or type 1 is a PDE1, PDE9, PDE10 or PDE11 inhibitor or a pharmaceutically acceptable salt thereof. PDE1, PDE9, PDE10 or PDE11 inhibitors which may be useful employed according to the present invention, can be found, for example, in US20020160939, WO03037432, US2004220186, WO2005003129, WO2005012485, WO2005120514 and WO03077949.

In another embodiment of the present invention the other active compound which is used in the treatment of diabetes mellitus type 2 and/or type 1 is an amylin agonist or a pharmaceutically acceptable salt thereof. A specific example of an amylin agonist includes, but is not limited to PRAMLINITIDE (CAS-No. 151126-32-8).

In another embodiment of the present invention the pharmaceutically acceptable salt of PRAMLINITIDE is the acetate salt of PRAMLINITIDE.

In another embodiment of the present invention the other active compound which is used in the treatment of diabetes mellitus type 2 and/or type 1 is cinnamon.

In another embodiment of the present invention the other active compound which is used in the treatment of diabetes mellitus type 2 and/or type 1 is a glucagon receptor antagonist or a pharmaceutically acceptable salt thereof. A specific example of a glucagons receptor antagonist includes, but is not limited to BAY-27-9955 (CAS-No. 202855-56-9).

In another embodiment of the present invention the other active compound which is used in the treatment of diabetes mellitus type 2 and/or type 1 is a glycogen-phosphorylase inhibitor or a pharmaceutically acceptable salt thereof. An example of a glycogen-phosphorylase inhibitor includes, but is not limited to INGLIFORIB (CAS-No. 186392-65-4).

In another embodiment of the present invention the other active compound which is used in the treatment of diabetes mellitus type 2 and/or type 1 is a fructose-1,6-bisphosphate inhibitor or a pharmaceutically acceptable salt thereof. An example of a fructose-1,6-bisphosphate inhibitor includes, but is not limited to MANAGLINAT DIALANETIL (= MB-06322; CAS-No. 280782-97-0) and MB-05032 (Cas-No. 261365-11-1).

In another embodiment of the present invention the pharmaceutically acceptable salt of MB-05032 is the hydrobromide salt of MB-05032.

In another embodiment of the present invention the other active compound which is used in the treatment of diabetes mellitus type 2 and/or type 1 is a cannabinoid (CB1) receptor antagonist or a pharmaceutically acceptable salt thereof. Specific examples of cannabinoid (CB1) receptor antagonists include, but are not limited to AVE-1625 (CAS-No. 261922-46-7), RIMONABANT (CAS-No. 168273-06-1) and SURINABANT (CAS-No. 288104-79-0).

In another embodiment of the present invention the pharmaceutically acceptable salt of RIMONABANT is the hydrochloride salt of RIMONABANT.

In another embodiment of the present invention the other active compound which is used in the treatment of diabetes mellitus type 2 and/or type 1 is an anti-obesity drug or a pharmaceutically acceptable salt thereof. Specific examples of anti-obesity drugs include, but are not limited to HMR-1426 (CAS-No. 262376-75-0), CETILISTAT (CAS-No. 282526-98-1) and SIBUTRAMINE (CAS-No. 106650-56-0).

In another embodiment of the present invention the pharmaceutically acceptable salt of HMR-1426 is the hydrochloride salt of HMR-1426. In another embodiment of the present invention the pharmaceutically acceptable salt of SIBUTRAMINE is the hydrochloride salt of SIBUTRAMINE.

More details with respect to preferred combination partners for Compound A are listed in Table 1:

**Table 1:**

| INN or Research Code | Structure/Chemical Name |
|---|---|
| BIM-51077 | L-histidyl-2-methylalanyl-L-glutamyl-glycyl-L-threonyl-L-phenylalanyl-L-threonyl-L-seryl-L-aspartyl-L-valyl-L-seryl-L-seryl-L-tyrosyl-L-leucyl-L-glutamyl-glycyl-L-glutaminyl-L-alanyl-L-alanyl-L-lysyl-L-glutamyl-L-phenylalanyl-L-isoleucyl-L-alanyl-L-tryptophyl-L-leucyl-L-valyl-L-lysyl-2-methylalanyl-L-argininamide |
| EXENATI DE | L-histidylglycyl-L-glutamylglycyl-L-threonyl-L-phenylalanyl-L-threonyl-L-seryl-L-aspartyl-L-leucyl-L-seryl-L-lysyl-glutaminyl-L-methionyl-L-glutamyl-L-glutamyl-L-glutamyl-L-alanyl-L-valyl-L-arginyl-L-leuycl-L-phenylalanyl-L-isoleucyl-L-glutamyl-L-tryptophyl-L-leucyl-L-lysyl-L-asparaginylglyclglycyl-L-prolyl-L-seryl-L-serylglycyl-L-alanyl-L-prolyl-L-prolyl-L-prolyl-L-serinamide |
| LIRAGLUTIDE | L-histidyl-L-alanyl-L-glutamyl-glycyl-L-threonyl-L-phenylalanyl-L-threonyl-L-seryl-L-aspartyl-L-valyl-L-seryl-L-seryl-L-tyrosyl-L-leucyl-L-glutamyl-glycyl-L-glutaminyl-L-alanyl-L-alanyl-Nepsilon-(Nalpha-hexadecanoyl-gamma-L-glutamyl)-L-lysyl-L-glutamyl-L-phenylalanyl-L-isoleucyl-L-alanyl-L-tryptophyl-L-leucyl-L-valyl-L-arginyl-glycyl-L-arginyl-glycine |
| ALBIGLUTIDE | HGEGTFTSDV SSYLEGQAAK EFIAWLVKGR HGEGTFTSDV SSYLEQAAK EFIAWLVKGR DAHKSEVAHR FKDLGEENFK ALVLIAFAQYLQQCPFEDHV KLVNEVTEFA KTCVADESAE NCDKSLHTLF GDKLCTVATLRETYGEMADC CAKQEPERNE CFLQHKDDNP NLPRLVRPEV DVMCTAFHDN EETFLKKLY EIARRHPYFY APELLFFAKR YKAAFTECCQ AADKAACLLP KLDELDEGK ASSAKQRLKC ASLQKFGERA FKAWAVARLS QRFPKAEFAEVSKLVTDLTK VHTECCHGDL LECADDRADL AKYICENQDS ISSKLKECCE KPLLEKSCI AEVENDEMPA DLPSLAADFV ESKDVCKNYA EAKDVFLGMF LYEARRHPD YSVVLLLRLA KTYETTLEKC CAAADPHECY AKVFDEFKPL VEEPQLIKQ NCELFEQLGE YKFQNALLVR YTKKVPQVST PTLVEVSRNLGKVGSKCCKH PEAKRMPCAE DYLSVVLNQL CVLHEKTPVS DRVTKCCTESLVNRRPCFSA LEVDETYVPK EFNAETFTFH ADICTLSEKE RQIKKQTALV ELVKHKKAT KEQLKAVMDD FAAFVEKCCK ADDKETCFAE EGKKLVAASQ AALGL |
| ZP-10 | H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-Lys-Lys-Lys-Lys-Lys-Lys-NH2 |
| TOLBUTAMIDE | 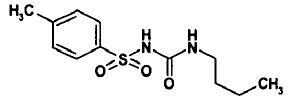 |
| | *N*-[(butylamino)carbonyl]-4-methylbenzenesulfonamide |
| TOLAZAMIDE | 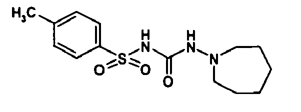 |
| | *N*-[(azepan-1-ylamino)carbonyl]-4-methylbenzenesulfonamide |
| GLIPIZIDE | 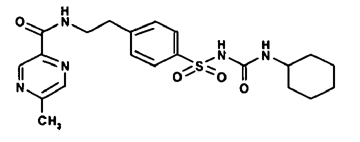 |
| | *N*-{2-[4-({[(cyclohexylamino)carbonyl]amino}sulfonyl)phenyl]ethyl}-5-methylpyrazine-2-carboxamide |
| CARBUTAMIDE | 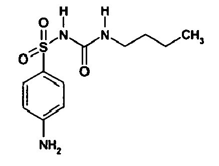 |
| | 4-amino-N-[(butylamino)carbonyl]benzenesulfonamide |
| GLISOXEPIDE | 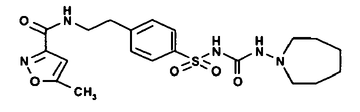 |
| | *N*-{2-[4-({[(azepan-1-ylamino)carbonyl]amino}sulfonyl)phenyl]ethyl}-5-methylisoxazole-3-carboxamide |
| GLISENTIDE | 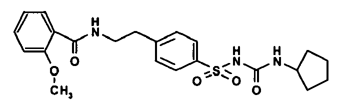 |
| | *N*-{2-[4-({[(cyclopentylamino)carbonyl]amino}sulfonyl)phenyl]ethyl}-2-methoxybenzamide |
| GLIBORNURIDE | 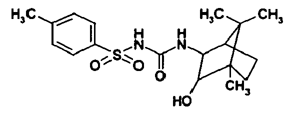 |
| | *N*-{[(3-hydroxy-4,7,7-trimethylbicyclo[2.2.1]hept-2-yl)amino]carbonyl}-4-methylbenzenesulfonamide |
| GLIBENCLAMIDE | 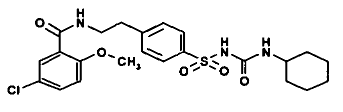 |
| | 5-chloro-*N*-{2-[4-({[(cyclohexylamino)carbonyl]amino}sulfonyl)phenyl]ethyl}-2-methoxybenzamide |
| GLIQUIDONE | 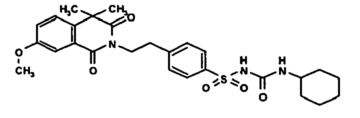 |
| | *N*-[(cyclohexylamino)carbonyl]-4-[2-(7-methoxy-4,4-dimethyl-1,3-dioxo-3,4-dihydroisoquinolin-2(1*H*)-yl)ethyl]benzenesulfonamide |
| GLIMEPIRIDE | 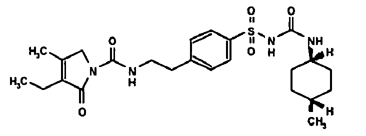 |
| | 3-ethyl-4-methyl-*N*-(2-{4-[({[(*trans*-4-methylcyclohexyl)amino]carbonyl}amino)sulfonyl]phenyl}ethyl)-2-oxo-2,5-dihydro-1*H*-pyrrole-1-carboxamide |
| GLICLAZIDE | 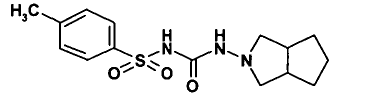 |
| | *N*-[(hexahydrocyclopenta[c]pyrrol-2(1H)-ylamino)carbonyl]-4-methylbenzenesulfonamide |
| METFORMIN | 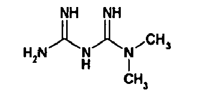 |
| | *N,N*-dimethylimidodicarbonimidic diamide |
| ACARBOSE | 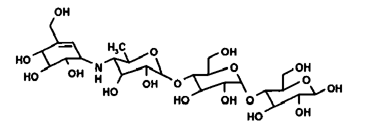 |
| | 4,6-dideoxy-4-{[4,5,6-trihydroxy-3-(hydroxymethyl)cyclohex-2-en-1-yl]amino}hexopyranosyl-(1→4)hexopyranosyl-(1→4)hexopyranose |
| MIGLITOL | 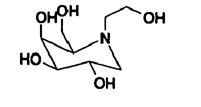 |
| | 1-(2-hydroxyethyl)-2-(hydroxymethyl)piperidine-3,4,5-triol |
| VOGLIBOSE | 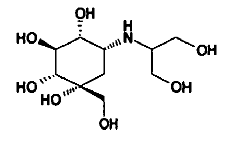 |
| | (1*R*,2*R*,3*S*,4*R*,5*R*)-5-{[2-hydroxy-1-(hydroxymethyl)ethyl]amino}-1-(hydroxymethyl)cyclohexane-1,2,3,4-tetrol |
| MURAGLITAZAR | 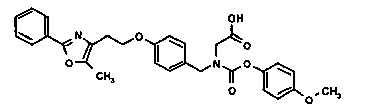 |
| | *N*-[(4-methoxyphenoxy)carbonyl]-*N*-{4-[2-(5-methyl-2-phenyl-1,3-oxazol-4-yl)ethoxy]benzyl}glycine |
| ROSIGLITAZONE | 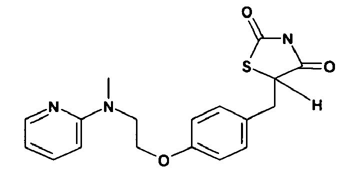 |
| | (5*RS*)-5-(4-{2-[methyl(pyridin-2-yl)amino]ethoxy}benzyl)-1, 3-thiazolidine-2,4-dione |
| PIOGLITAZONE | 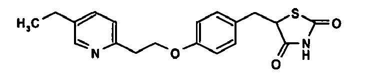 |
| | 5-{4-[2-(5-ethylpyridin-2-yl)ethoxy]benzyl}-1,3-thiazolidine-2,4-dione |
| FARGLITAZAR | 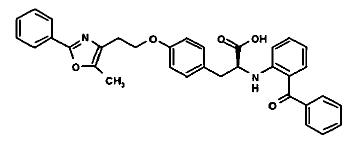 |
| | *N*-(2-benzoylphenyl)-O-[2-(5-methyl-2-phenyl-1,3-oxazol-4-yl)ethyl]-L-tyrosine |
| NAVEGLITAZAR | 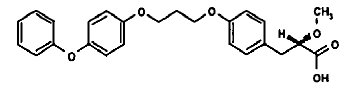 |
| | (2*S*)-2-methoxy-3-{4-[3-(4-phenoxyphenoxy)propoxy]phenyl}propanoic acid |
| NETOGLITAZONE | 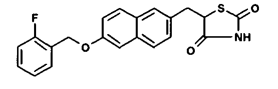 |
| | 5-({6-[(2-fluorobenzyl)oxy]-2-naphthyl}methyl)-1,3-thiazolidine-2,4-dione |
| RIVOGLITAZONE | 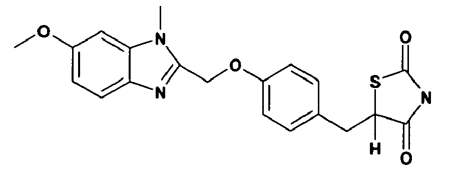 |
| | (5*R*)-5-{4-[(6-methoxy-1-methyl-1*H*-benzimidazol-2-yl)methoxy]benzyl}-1,3-thiazolidine-2,4-dione |
| K-111 | 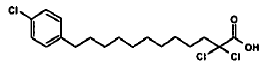 |
| | 2,2-dichloro-12-(4-chlorophenyl)dodecanoic acid |
| SODELGLITAZAR=GW-677954 | 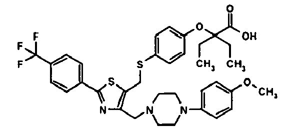 |
| | 2-ethyl-2-(4-{[(4-{[4-(4-methoxyphenyl)piperazin-1-yl]methyl}-2-[4-(trifluoromethyl)phenyl]-1,3-thiazol-5-yl)methyl]thio}phenoxy)butanoic acid |
| (-)-Halofenate | 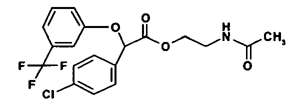 |
| | (-)-2-Acetamidoethyl 4-chlorophenyl(3-trifluoromethylphenoxy)acetate |
| REPAGLINIDE | 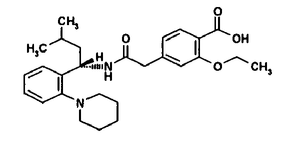 |
| | 2-ethoxy-4-(2-{[(1*S*)-3-methyl-1-(2-piperidin-1-ylphenyl)butyl]amino}-2-oxoethyl)benzoic acid |
| NATEGLINIDE | 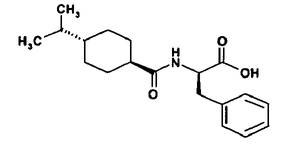 |
| | *N*-[(*trans*-4-isopropylcyclohexyl)carbonyl]-D-phenylalanine |
| MITIGLINIDE | 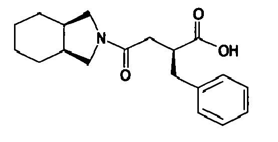 |
| | (2*S*)-2-benzyl-4-[(3a*R*,7a*S*)-octahydro-2H-isoindol-2-yl]-4-oxobutanoic acid |
| SITAGLIPTIN | 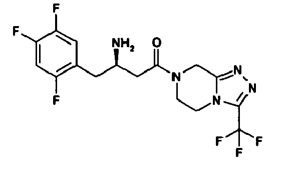 |
| | (2*R*)-4-oxo-4-[3-(trifluoromethyl)-5,6-dihydro[1,2,4]triazolo[4,3-*a*]pyrazin-7(8*H*)-yl]-1-(2 ,4, 5-trifluorophenyl)butan-2-amine |
| SAXAGLIPTIN | 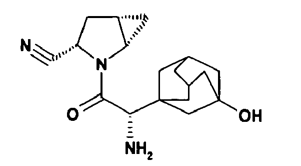 |
| | (1*S*,3*S*,5*S*)-2-[(2*S*)-2-amino-2-(3-hydroxy-1-adamantyl)acetyl]-2-azabicyclo[3.1.0]hexane-3-carbonitrile |
| VILDAGLIPTIN | 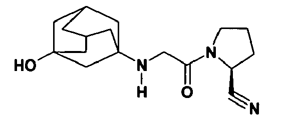 |
| | (2*S*)-1-[*N*-(3-hydroxy-1-adamantyl)glycyl]pyrrolidine-2-carbonitrile |
| DENAGLIPTIN | 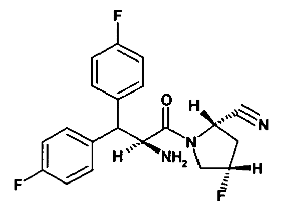 |
| | (2*S*,4*S*)-4-fluoro-1-[4-fluoro-β-(4-fluorophenyl)-L-phenylalanyl]pyrrolidine-2-carbonitrile |
| ALOGLIPTIN | 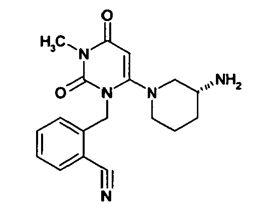 |
| | 2-({6-[(3*R*)-3-aminopiperidin-1-yl]-3-methyl-2,4-dioxo-3,4- dihydropyrimidin-1(2*H*)-yl}methyl)benzonitrile |
| P32/98 | 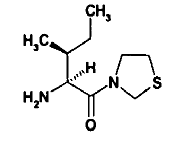 |
| | (2*S*,3*S*)-3-methyl-1-oxo-1-(1,3-thiazolidin-3-yl)pentan-2-amine |
| SILDENAFIL | 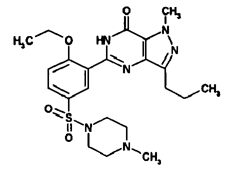 |
| | 5-{2-ethoxy-5-[(4-methylpiperazin-1-yl)sulfonyl]phenyl}-1-methyl-3-propyl-1,6-dihydro-7*H*-pyrazolo[4,3-*d*]pyrimidin-7-one |
| VARDENAFIL | 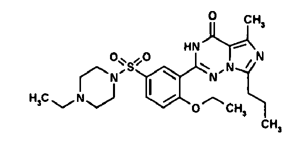 |
| | 2-{2-ethoxy-5-[(4-ethylpiperazin-1-yl)sulfonyl]phenyl}-5-methyl-7-propylimidazo[5,1-*f*][1,2,4]triazin-4(3*H*)-one |
| TADALAFIL | 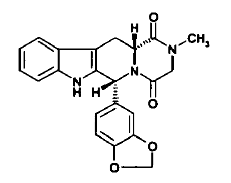 |
| | (6*R*,12a*R*)-6-(1,3-benzodioxol-5-yl)-2-methyl-2,3,6,7,12,12a-hexahydropyrazino[1',2':1,6]pyrido[3,4-*b*]indole-1,4-dione |
| UDENAFIL | 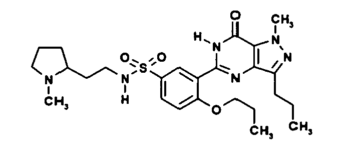 |
| | 3-(1-methyl-7-oxo-3-propyl-6,7-dihydro-1*H*-pyrazolo[4,3-*d*]pyrimidin-5-yl)-*N*-[2-(1-methylpyrrolidin-2-yl)ethyl]-4-propoxybenzenesulfonamide |
| AVANAFIL | 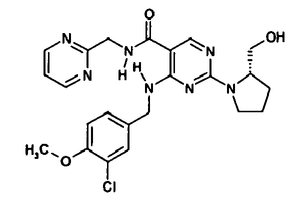 |
| | 4-[(3-chloro-4-methoxybenzyl)amino]-2-[(2*S*)-2-(hydroxymethyl)pyrrolidin-1-yl]-*N*-(pyrimidin-2-ylmethyl)pyrimidine-5- carboxamide |
| PRAMLINTIDE | L-lysyl-L-cysteinyl-L-asparaginyl-L-threonyl-L-alanyl-L-threonyl-L-cysteinyl-L-alanyl-L-threonyl-L-glutaminyl-L-arginyl-L-leucyl-L-alanyl-L-asparaginyl-L-phenylalanyl-L-leucyl-L-valyl-L-histidyl-L-seryl-L-seryl-L-asparaginyl-L-asparaginyl-L-phenylalanylglycyl-L-prolyl-L-isoleucyl-L-leucyl-L-prolyl-L-prolyl-L-threonyl-L-asparaginyl-L- valylglycyl-L-seryl-L-asparaginyl-L-threonyl-L-tyrosinamide, cyclic (2->7)disulfide |
| BAY-27-9955 | 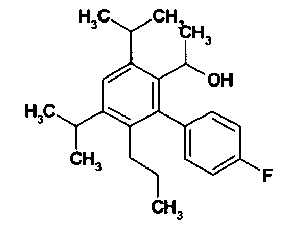 |
| | 2-(4'-fluoro-3,5-diisopropyl-6-propylbiphenyl-2-yl)ethanol |
| INGLIFORIB | 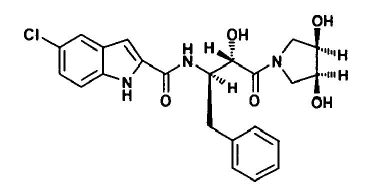 |
| | *N*-{(1*S*,2*R*)-1-benzyl-3-[(3*R*,4*S*)-3,4-dihydroxypyrrolidin-1-yl]-2-hydroxy-3-oxopropyl}-5-chloro-1*H*-indole-2-carboxamide |
| MANAGLINAT DIALANTENIL = MB-06322 | 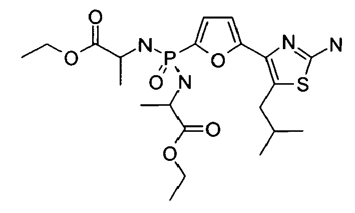 |
| | ethyl 4-[5-(2-amino-5-isobutyl-1,3-thiazol-4-yl)-2-furyl]-2,6-dimethyl-7-oxo-8-oxa-3,5-diaza-4-phosphadecan-1-oate 4-oxide |
| MB-05032 | 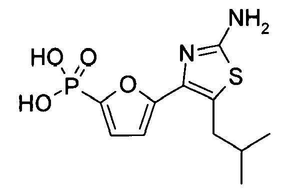 |
| | [5-(2-amino-5-isobutyl-1,3-thiazol-4-yl)-2-furyl]phosphonic acid |
| AVE-1625 | 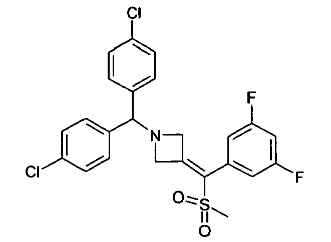 |
| | 1-[bis(4-chlorophenyl)methyl]-3-[(3,5-difluorophenyl)(methylsulfonyl)methylene]azetidine |
| RIMONABANT | 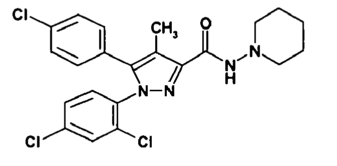 |
| | 5-(4-chlorophenyl)-1-(2,4-dichlorophenyl)-4-methyl-*N*-piperidin-1-yl-1*H*-pjrrazole-3-carboxamide |
| SURINABANT | 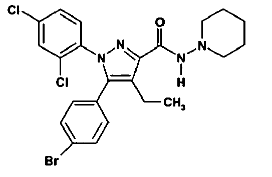 |
| | 5-(4-bromophenyl)-1-(2,4-dichlorophenyl)-4-ethyl-*N*-piperidin-1-yl-1*H*-pyrazole-3-carboxamide |
| HMR-1426 | 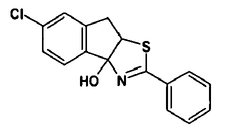 |
| | 6-chloro-2-phenyl-8,8a-dihydro-3a*H*-indeno[1,2-*d*][1,3]thiazol-3a-ol |
| CETILISTAT | 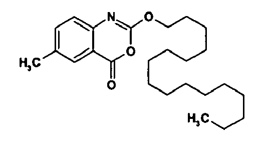 |
| | 2-(hexadecyloxy)-6-methyl-4*H*-3,1-benzoxazin-4-one |
| SIBUTRAMINE | 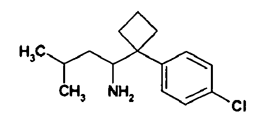 |
| | 1-[1-(4-chLorophenyl)cyclobutyl]-3-methylbutan-1-amine |

Additional information with regard to the preparation, suitable dosage forms and dose ranges of the glucagon-like-peptide-1 receptor agonists listed in Table 1 can be found in the following patents/patent applications: WO0334331, EP0981611, WO9808871, WO0104156 and W003059934.

The sulfonylurea agents TOLBUTAMIDE, TOLAZAMIDE, GLIPIZIDE, CARBUTAMIDE, GLISOXEPIDE; GLISENTIDE, GLIBORNURIDE, GLIBENCLAMIDE, GLIQUIDONE, GLIMEPIRIDE and GLICLAZIDE listed in Table 1 are commercially available. The person skilled in the art is familiar with suitable formulations and dose ranges of these compounds.

The biguanide agent METFORMIN listed in Table 1 is commercially available. The person skilled in the art is familiar with suitable formulations and dose ranges of this compound.

The alpha-glucosidase inhibitors ACARBOSE, MIGLITOL and VOGLIBOSE listed in Table 1 are commercially available. The person skilled in the art is familiar with suitable formulations and dose ranges of this compound.

Additional information with regard to the preparation, suitable dosage forms and dose ranges of the PPAR-agonists listed in Table 1 can be found in the following patents/patent applications: WO0121602, EP03306228, EP0658161, EP0193256, W09731907, WO0140169, W002100813, EP0604983, EP0745600, WO9615784, WO0259098 and EP1183020.

The metiglinide agents REPAGLINIDE, NATEGLINIDE and MITIGLINIDE listed in Table 1 are commercially available. The person skilled in the art is familiar with suitable formulations and dose ranges of this compound.

Additional information with regard to the preparation, suitable dosage forms and dose ranges of the DPP IV inhibitors listed in Table 1 can be found in the following patents/patent applications: WO03004498, W00168603, WO0034241, WO0302531, WO9961431 and WO2005095381.

Additional information with regard to the preparation, suitable dosage forms and dose ranges of the PDE5 inhibitors listed in Table 1 can be found in the following patents/patent applications: WO0213798, WO0260422, WO2004082667, WO0027848 and EP1219609.

Additional information with regard to the preparation, suitable dosage forms and dose ranges of the amylin analogue PRAMLINTIDE listed in Table 1 can be found in EP0567626.

Additional information with regard to the preparation, suitable dosage forms and dose ranges of the glucagon receptor antagonist listed in Table 1 can be found in WO9804528.

Additional information with regard to the preparation, suitable dosage forms and dose ranges of the glycogen-phosphorylase inhibitor listed in Table 1 can be found in WO9639385.

Additional information with regard to the preparation, suitable dosage forms and dose ranges of the fructose-1,6-bisphosphate inhibitors can be found in WO0001495 and WO0147935.

Additional information with regard to the preparation, suitable dosage forms and dose ranges of the cannabinoid (CB1) receptor antagonist listed in Table 1 can be found in EP1112251, EP0576357 and WO0046209.

Additional information with regard to the preparation, suitable dosage forms and dose ranges of HMR-1426, CETILISTAT and SIBUTRAMINE listed in Table 1 can be found in the following patents/patent applications: WO0018749, EP1144395 and EP0397831.

"Pharmaceutically acceptable salts" of Compound A or the other active compound(s) which is (are) used in the treatment of diabetes mellitus type 2 and/or type 1 are not limited to the specific examples given above. The term refers to non-toxic salts of these compounds. These pharmaceutically acceptable salts are generally prepared by reacting a free base with a suitable organic or inorganic acid or by reacting an acid with a suitable organic or inorganic base. Particular mention may be made of the pharmaceutically acceptable inorganic and organic acids customarily used in pharmacy. Those suitable are in particular water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)-benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methanesulfonic acid or 1-hydroxy-2-naphthoic acid. As examples of pharmaceutically acceptable salts with bases may be mentioned the lithium, sodium, potassium, calcium, aluminium, magnesium, titanium, ammonium, meglumine or guanidinium salts.

It is understood that Compound A and the other active compound(s) which is (are) used in the treatment of diabetes mellitus type 2 and/or type 1 and their pharmaceutically acceptable salts can also be present in the form of their pharmaceutically acceptable solvates, and in particular in the form of their hydrates.

The combinations according to the invention may be administered by any suitable route, for example, by the oral, sublingual, buccal, intravenous, intraarterial, intramuscular, subcutaneous, intracutaneous, topical, transdermal, intranasal, intraperitoneal, rectal or vaginal route, by inhalation or by insufflation.

Tablets, coated tablets (dragees), pills, cachets, capsules (caplets), granules, solutions, emulsions and suspensions are e.g. suitable for oral administration. In particular, said formulations can be adapted so as to represent, for example, an enteric form, an immediate release form, a delayed release form, a repeated dose release form, a prolonged release form or a sustained release form. Said forms can be obtained, for example, by coating tablets, by dividing tablets into several compartments separated by layers disintegrating under different conditions (e.g. pH conditions) or by coupling the active compound to a biodegradable polymer.

Administration by inhalation is preferably made by using an aerosol. The aerosol is a liquid-gaseous dispersion, a solid-gaseous dispersion or a mixed liquid/solid-gaseous dispersion.

The aerosol may be generated by means of aerosol-producing devices such as dry powder inhalers (DPIs), pressurized metered dose inhalers (PMDIs) and nebulizers. Depending on the kind of the active compound to be administered, the aerosol-producing device can contain the active compound in form of a powder, a solution or a dispersion. The powder may contain, for example, one or more of the following auxiliaries: carriers, stabilizers and fillers. The solution may contain in addition to the solvent, for example, one or more of the following auxiliaries: propellants, solubilizers (co-solvents), surfactants, stabilizers, buffers, tonicity adjusting agents, preservatives and flavorings. The dispersion may contain in addition to the dispersant, for example, one or more of the following auxiliaries: propellants, surfactants, stabilizers, buffers, preservatives and flavorings. Examples of carriers include, but are not limited to, saccharides, e.g. lactose and glucose. Examples of propellants include, but are not limited to, fluorohydrocarbons, e.g. 1,1,1,2-tetrafluoroethane and 1,1,1,2,3,3,3-heptafluoropropane.

The particle size of the aerosol particles (solid, liquid or solid/liquid particles) is preferably less than 100 µm, more preferably it is in the range of from 0.5 to 10 µm, in particular in the range of from 2 to 6 µm (D50 value, measured by laser diffraction).

For parenteral modes of administration such as, for example, intravenous, intraarterial, intramuscular, subcutaneous, intracutaneous and intraperitoneal administration, preferably solutions (e.g. sterile solutions, isotonic solutions) are used. They are preferably administered by injection or infusion techniques.

The pharmaceutical compositions (formulations) comprising Compound A or a pharmaceutically acceptable salt thereof and/or one or two other active compound(s) or pharmaceutically acceptable salt(s) thereof which is (are) used in the treatment of diabetes mellitus type 2 and/or type 1 and at least one pharmaceutically acceptable auxiliary can be manufactured in a manner known to a person skilled in the art, e.g. by dissolving, mixing, granulating, dragee-making, levigating, emulsifying, encapsulating, entrapping or lyophilizing processes.

As pharmaceutically acceptable auxiliaries, any auxiliaries known to be suitable for preparing pharmaceutical compositions (formulations) can be used. Examples thereof include, but are not limited to, solvents, excipients, dispersants, emulsifiers, solubilizers, gel formers, ointment bases, antioxidants, preservatives, stabilizers, carriers, fillers, binders, thickeners, complexing agents, disintegrating agents, buffers, permeation promoters, polymers, lubricants, coating agents, propellants, tonicity adjusting agents, surfactants, colorants, flavorings, sweeteners and dyes. In particular, auxiliaries of a type appropriate to the desired formulation and the desired mode of administration are used.

The preferred mode of administration of the combinations according to the invention depend on the specific combination partners.

As mentioned above Compound A or a pharmaceutically acceptable salt of either may be administered in a variety of forms. These include, for example, liquid, semi-solid and solid dosage forms, such as liquid solutions (e.g., injectable and infusible solutions), dispersions or suspensions, tablets, pills, powders, liposomes or suppositories. The preferred form depends on the intended mode of administration and the combination partner.

The most preferred mode of administration of Compound A or a pharmaceutically acceptable salt of either is oral. In another preferred embodiment Compound A or a pharmaceutically acceptable salt of either is administered by intravenous infusion or injection. In a further embodiment Compound A or a pharmaceutically acceptable salt of either is administered by intramuscular or subcutaneous injection. Other routes of administration are also contemplated, including for example intranasal and transdermal routes, and by inhalation.

The preferred mode of administration of the other active compound(s) which is (are) used in combination with Compound A or a pharmaceutically acceptable salt of either depends on the specific agent.

EXENATIDE, BIM-51077, ALBIGLUTIDE, ZP-10 or PRAMLINTIDE, for example, are preferably administered via subcutaneous injection. The preferred mode of administration of compounds like TOLBUTAMIDE, TOLAZAMIDE, GLIPIZIDE, CARBUTAMIDE, GLISOXEPIDE, GLISENTIDE, GLIBORNURIDE, GLIBENCLAMIDE, GLIQUIDONE, GLIMEPIRIDE, GLICLAZIDE, METFORMIN, ACARBOSE, MIGLITOL, VOGLIBOSE, ROSIGLITAZONE, PIOGLITAZONE, FARGLITAZAR, NAVEGLITAZAR, NETOGLITAZONE, RIVOGLITAZONE, K-111, GW-677954, (-)-HALOFENATE, REPAGLINIDE, NATEGLINIDE, MITIGLINIDE, SITAGLIPTIN, SAXAGLIPTIN, VILDAGLIPTIN, DENAGLIPTIN, ALOGLIPTIN, P32/98, SILDENAFIL; VARDENAFIL, TADALAFIL, UDENAFIL, AVANAFIL, BAY-27-9955, INFLIGORIB, MB-06322, MB-05022, AVE-1625, RIMONABANT, SURINABANT, HMR-1426, CETILISTAT, SIBUTRAMINE and cinnamon is oral. Further information with regard to the preferred mode of administration of the other active agent(s) which is (are) used in combination with Compound A or a pharmaceutically acceptable salt of either is summarized in Table 2 below.

As part of the combination therapy according to the invention Compound A or a pharmaceutically acceptable salt thereof and the one or two other active compound(s) or pharmaceutically acceptable salt(s) thereof which is (are) used in the treatment of diabetes mellitus type 2 and/or type 1 are dosed in an order of magnitude customary for the mono-therapy, it more likely being possible, on account of the individual actions, which are mutually positively influencing and reinforcing, to reduce the respective doses on the combined administration of Compound A or a pharmaceutically acceptable salt thereof and the one or two other active compound(s) which is (are) used in the treatment of diabetes mellitus type 2 and/or type 1 with the norm.

In the case of oral administration of Compound A the daily dose for an adult patient for the mono-therapy is in the range from 0.2 to 30 mg per day, preferably in the range of 0.2 to 10 mg per day, more preferably in the range of 0.5 to 5 mg per day.

Further information with regard to the preferred routes of administration and typical dosages (for mono-therapy) of the other active compound(s) which is (are) used in combination with Compound A is summarized in Table 2.

The proportions in which Compound A or a pharmaceutically acceptable salt thereof and the other active compound(s) or a pharmaceutically acceptable salt thereof may be used in the pharmaceutical compositions, combinations products or kits according to the invention are variable. Depending on the choice of the Compound A or a pharmaceutically acceptable salt thereof and the choice of the other active compound(s) or a pharmaceutically acceptable salt thereof, the weight ratios which may be used within the scope of the present invention vary on the basis of the different molecular weights of the various compounds and their different potencies.

As a general rule, without intended to be limiting, the pharmaceutical compositions, combination products or kits according to the invention may contain
Compound A and BIM-51077 in ratios by weight ranging from 50:1 to 1:2, preferably from 20:1 to 1:1;
Compound A and EXENATIDE in ratios by weight ranging from 500:1 to 25:1, preferably from 200:1 to 50:1;
Compound A and LIRAGLUTIDE in ratios by weight ranging from 10:1 to 1:1, preferably from 5:1 to 2:1;
Compound A and TOLBUTAMIDE in ratios by weight ranging from 1:4000 to 1:100, preferably from 1:1000 to 1:250;
Compound A and TOLAZAMIDE in ratios by weight ranging from 1:300 to 1:20, preferably from 1:150 to 1:50;
Compound A and GLIPIZIDE in ratios by weight ranging from 1:80 to 1:1, preferably from 1:40 to 1:2;
Compound A and GLISOXEPIDE in ratios by weight ranging from 1:32 to 2:1, preferably from 1:10 to 1:1;
Compound A and GLIBORNURIDE in ratios by weight ranging from 1:150 to 1:3; preferably from 1:50 to 1:6;
Compound A and GLIBENCLAMIDE in ratios by weight ranging from 1:20 to 3:1, preferably from 1:5 to 1:1;
Compound A and GLIQUIDONE in ratios by weight ranging from 1:250 to 1:3, preferably from 1:60 to 1:6;
Compound A and GLIMEPIRIDE in ratios by weight ranging from 1:12 to 5:1, preferably from 1:4 to 2:1;
Compound A and GLICLAZIDE in ratios by weight ranging from 1:250 to 1:6, preferably from 1:60 to 1:15;
Compound A and METFORMIN in ratios by weight ranging from 1:8000 to 1:200, preferably from 1:2000 to 1:500;
Compound A and ACARBOSE in ratios by weight ranging from 1:1200 to 1:30, preferably from 1:300 to 1:75;
Compound A and MIGLITOL in ratios by weight ranging from 1:600 to 1:30, preferably from 1:150 to 1:75;
Compound A and VOGLIBOSE in ratios by weight ranging from 5:1 to 1:2, preferably from 2:1 to 1:1;
Compound A and MURAGLITAZAR in ratios by weight ranging from 1:10 to 2:1, preferably from 1:5 to 1:1;
Compound A and ROSIGLITAZONE in ratios by weight ranging from 1:16 to 1:1, preferably from 1:8 to 1:2;
Compound A and PIOGLITAZONE in ratios by weight ranging from 1:90 to 1:3, preferably from 1:45 to 1:8;
Compound A and FARGLITAZAR in ratios by weight ranging from 1:20 to 10:1, preferably from 1:10 to 4:1;
Compound A and NAVEGLITAZAR in ratios by weight ranging from 50:1 to 1:2, preferably from 20:1 to 1:1;
Compound A and NETOGLITAZONE in ratios by weight ranging from 1:100 to 5:1, preferably from 1:50 to 2:1;
Compound A and RIVOGLTAZONE in ratios by weight ranging from 1:20 to 5:1, preferably from 1:10 to 2:1;
Compound A and K-111 in ratios by weight ranging from 1:40 to 1:2, preferably from 1:20 to 1:5;
Compound A and GW-677954 in ratios by weight ranging from 1:20 to 2:1, preferably from 1:10 to 1:1;
Compound A and (-)-HALOFENATE in ratios by weight ranging from 1:2000 to 1:200, prteferably from 1:1000 to 1:500;
Compound A and REPAGLINIDE in ratios by weight ranging from 1:32 to 10:1, preferably from 1:16 to 4:1;
Compound A and NATEGLINIDE in ratios by weight ranging from 1:1000 to 1:40, preferably from 1:500 to 1:100;
Compound A and MITIGLINIDE in ratios by weight ranging from 1:250 to 1:8, preferably from 1:120 to 1:20;
Compound A and SITAGLIPTIN in ratios by weight ranging from 1:200 to 1:20, preferably from 1:100 to 1:50;
Compound A and SAXAGLIPTIN in ratios by weight ranging from 1:20 to 1:2; preferably from 1:10 to 1:2;
Compound A and VILDAGLIPTIN in ratios by weight ranging from 1:200 to 1:5, preferably from 1:100 to 1:10;
Compound A and DENAGLIPTIN in ratios by weight ranging from 1:100 to 1:1, preferably from 1:50 to 1:2;
Compound A and ALOGLIPTIN in ratios by weight ranging from 1:100 to 1:2, preferably from 1:50 to 1:5;
Compound A and P32/98 in ratios by weight ranging from 1:200 to 1:6, preferably from 1:100 to 1:15;

Compound A and SILDENAFIL in ratios by weight ranging from 1:200 to 1:10, preferably from 1:100 to 1:25;
Compound A and VARDENAFIL in ratios by weight ranging from 1:40 to 2:1, preferably from 1:20 to 1:1;
Compound A and TADALAFIL in ratios by weight ranging from 1:40 to 1:2, preferably from 1:20 to 1:5;
Compound A and UDENAFIL in ratios by weight ranging from 1:400 to 1:20, preferably 1:200 to 1:50;
Compound A and AVANAFIL in ratios by weight ranging from 1:600 to 1:10 , preferably from 1:300 to 1:25;
Compound A and PRIMLINTIDE in ratios by weight ranging from 250:1 to 5:1, preferably from 100:1 to 10:1;
Compound A and cinnamon in ratios by weight ranging from 1:12000 to 1:200, preferably from 1:6000 to 1:500;
Compound A and BAY-27-9955 in ratios by weight ranging from 1:400 to 1:10, preferably from 1:2000 to 1:25;
Compound A and INGLIFORIB in ratios by weight ranging from 1:100 to 1:4, preferably from 1:50 to 1:10;
Compound A and MB-06322 in ratios by weight ranging from 1:1600 to 1:40, preferably from 1:800 to 1:100;
Compound A and AVE-1625 in ratios by weight ranging from 1:20 to 1:2, preferably from 1:10 to 1:5;
Compound A and RIMONABANT in ratios by weight ranging from 1:40 to 1:4, preferably from 1:20 to 1:10;
Compound A and SURINABANT in ratios by weight ranging from 1 :20 to 2:1, preferably from 1:10 to 1:1;
Compound A and CETILISTAT in ratios by weight ranging from 1:1800 to 1:25, preferably from 1:900 to 1:60;
Compound A and SIBUTRAMINE in ratios by weight ranging from 1:30 to 1:2, preferably from 1:15 to 1:5;
Corresponding ratios likely may be used in the triple combinations according to the invention.

**Table 2: Preferred routes of administration and dosages:**

| INN or Research Code | Preferred Treatment of | Preferred route of Administration | Typical Daily dose (dose ranges) used for mono-therapy |
|---|---|---|---|
| Insulin/Insulin analogs | Diabetes mellitus type 1 | subcutaneous injection | On demand |
| | Diabetes mellitus type 2 | | |
| EXUBERA | Diabetes mellitus type 1 | Inhalation | On demand |
| | Diabetes mellitus type 2 | | |
| BIM-51077 | Diabetes mellitus type 2 | subcutaneous injection | 100 - 800 µg |
| EXENATIDE | Diabetes mellitus type 2 | subcutaneous injection | 10-20 µg |
| LIRAGLUTIDE | Diabetes mellitus type 2 | subcutaneous injection | 0.5 - 2 mg |
| ALBIGLUTIDE | Diabetes mellitus type 2 | subcutaneous injection | 2-20 µg |
| ZP-10 | Diabetes mellitus type 2 | subcutaneous injection | |
| TOLBUTAMIDE | Diabetes mellitus type 2 | oral | 0.5 to 2.0 g |
| TOLAZAMIDE | Diabetes mellitus type 2 | oral | 100 to 150 mg |
| GLIPIZIDE | Diabetes mellitus type 2 | oral | 5 to 40 mg, preferably 5 to 20 mg |
| CARBUTAMIDE | Diabetes mellitus type 2 | oral | up to 17 mg/kg |
| GLISOXEPIDE | Diabetes mellitus type 2 | oral | 2 to 16 mg |
| GLISENTIDE | Diabetes mellitus type 2 | oral | |
| GLIBORNURIDE | Diabetes mellitus type 2 | oral | 12.5 to 75 mg |
| GLIBENCLAMIDE | Diabetes mellitus type 2 | oral | 1.75 to 10.5 mg |
| GLIQUIDONE | Diabetes mellitus type 2 | oral | 15 to 120 mg |
| GLIMEPIRIDE | Diabetes mellitus type 2 | oral | 1 to 6 mg |
| GLICLAZIDE | Diabetes mellitus type 2 | oral | 30 to 120 mg |
| METFORMIN | Diabetes mellitus type 2 | oral | 1000 to 3800 mg |
| ACARBOSE | Diabetes mellitus type 2 | oral | 150 to 600 mg, preferably 150 to 300 mg |
| MIGLITOL | Diabetes mellitus type 2 | oral | 150 to 300 mg |
| VOGLIBOSE | Diabetes mellitus type 2 | oral | 0.6 to 0.9 mg |
| MURAGLITAZAR | Diabetes mellitus type 2 | oral or by injection | 2.5 to 5 mg |
| ROSIGLITAZONE | Diabetes mellitus type 2 | oral | 4 to 8 mg |
| PIOGLITAZONE | Diabetes mellitus type 2 | oral | 15 to 45 mg |
| FARGLITAZAR | Diabetes mellitus type 2 | oral | 0.5 to 10 mg |
| NAVEGLITAZAR | Diabetes mellitus type 2 | oral | 0.004 to 1.2 mg |
| NETOGLITAZONE | Diabetes mellitus type 2 | oral | 1-50 mg |
| RIVOGLITAZONE | Diabetes mellitus type 2 | oral | 1-10 mg |
| K-111 | Diabetes mellitus type 2 | oral | 10 to 20 mg |
| GW-677954 | Diabetes mellitus type 2 | oral | 2.5 to 20 mg |
| (-)-Halofenate | Diabetes mellitus type 2 | oral | ≅ 1000 mg |
| REPAGLINIDE | Diabetes mellitus type 2 | oral | 0.5 to 16 mg |
| NATEGLINIDE | Diabetes mellitus type 2 | oral | 180 to 540 mg |
| MITIGLINIDE | Diabetes mellitus type 2 | oral | 40 mg/meal |
| SITAGLIPTIN | Diabetes mellitus type 2 | oral | ≅ 100 mg |
| SAXAGLIPTIN | Diabetes mellitus type 2 | oral | ≅ 10 mg |
| VILDAGLIPTIN | Diabetes mellitus type 2 | oral | 25-100 mg |
| DENAGLIPTIN | Diabetes mellitus type 2 | oral | 5-50 mg |
| ALOGLIPTIN | Diabetes mellitus type 2 | oral | 10-50 mg |
| P32/98 | Diabetes mellitus type 2 | oral | 30 - 100 mg |
| SILDENAFIL | Diabetes mellitus type 2 | oral | 50 to 100 mg |
| | Diabetes mellitus type 1 | | |
| VARDENAFIL | Diabetes mellitus type 2 | oral | 2.5 to 20 mg |
| | Diabetes mellitus type 1 | | |
| TADALAFIL | Diabetes mellitus type 2 | oral | 10 to 20 mg |
| | Diabetes mellitus type 1 | | |
| UDENAFIL | Diabetes mellitus type 2 | oral | 100-200 mg |
| | Diabetes mellitus type 1 | | |
| AVANAFIL | Diabetes mellitus type 2 | oral | 50-300 mg |
| | Diabetes mellitus type 1 | | |
| PRAMLINTIDE | Diabetes mellitus type 2 | subcutaneous injection | 20 to 120 µg |
| | Diabetes mellitus type 1 | | |
| cinnamon | Diabetes mellitus type 2 | oral | 1 to 6 g |
| BAY-27-9955 | Diabetes mellitus type 2 | oral | 50 to 200 mg |
| INGLIFORIB | Diabetes mellitus type 2 | oral | 20 to 50 mg |
| MB-06322 | Diabetes mellitus type 2 | oral | 200 to 800 mg |
| MB-05032 | Diabetes mellitus type 2 | oral | |
| AVE-1625 | Diabetes mellitus type 2 | oral | ≅ 10 mg |
| RIMONABANT | Diabetes mellitus type 2 | oral | 20 mg |
| SURINABANT | Diabetes mellitus type 2 | oral | 3 to 10 mg |
| HMR-1426 | Diabetes mellitus type 2 | oral | |
| CETILISTAT | Diabetes mellitus type 2 | oral | 120 to 920 mg |
| SIBUTRAMINE | Diabetes mellitus type 2 | oral | 10 to 15 mg |

### Examples

**Table 3: Preferred combinations**

| **Example Number** | **Combination** | |
|---|---|---|
| **1** | Compound A* | human insulin |
| **2** | Compound A* | Insulin analogue |
| **3** | Compound A* | BIM-51077 |
| **4** | Compound A* | EXENATIDE |
| **5** | Compound A* | LIRAGLUTIDE |
| **6** | Compound A* | ALBIGLUTIDE |
| **7** | Compound A* | ZP-10 |
| **8** | Compound A* | TOLBUTAMIDE |
| **9** | Compound A* | TOLBUTAMIDE sodium |
| **10** | Compound A* | TOLAZAMIDE |
| **11** | Compound A* | GLIPIZIDE |
| **12** | Compound A* | CARBUTAMIDE |
| **13** | Compound A* | GLISOXEPIDE |
| **14** | Compound A* | GLISENTIDE |
| **15** | Compound A* | GLIBORNURIDE |
| **16** | Compound A* | GLIBENCLAMIDE |
| **17** | Compound A* | GLIQUIDONE |
| **18** | Compound A* | GLIQUIDONE sodium |
| **19** | Compound A* | GLIMEPIRIDE |
| **20** | Compound A* | GLICLAZIDE |
| **21** | Compound A* | METFORMIN |
| **22** | Compound A* | METFORMIN hydrochloride |
| **23** | Compound A* | ACARBOSE |
| **24** | Compound A* | MIGLITOL |
| **25** | Compound A* | VOGLIBOSE |
| **26** | Compound A* | MURAGLITAZAR |
| **27** | Compound A* | ROSIGLTAZONE |
| **28** | Compound A* | ROSIGLITAZONE maleate |
| **29** | Compound A* | PIOGLITAZONE |
| **30** | Compound A* | PIOGLITAZONE dihydrochloride |
| **31** | Compound A* | FARGLITAZAR |
| **32** | Compound A* | NAVEGLITAZAR |
| **33** | Compound A* | NETOGLITAZONE |
| **34** | Compound A* | NETOGLlTAZONE |
| **35** | Compound A* | RIVOGLTAZONE hydrochloride |
| **36** | Compound A* | K-111 |
| **37** | Compound A* | K-111 sodium |
| **38** | Compound A* | GW-677954 |
| **39** | Compound A* | (-)-Halofenate |
| **40** | Compound A* | REPAGLINIDE |
| **41** | Compound A* | NATEGLINIDE |
| **42** | Compound A* | MITIGLINIDE |
| **42** | Compound A* | MITIGLINIDE potassium |
| **44** | Compound A* | MITIGLINIDE calcium |
| **45** | Compound A* | SITAGLIPTIN |
| **46** | Compound A* | SITAGLIPTIN phosphate |
| **48** | Compound A* | VILDAGLIPTIN |
| **49** | Compound A* | DENAGLIPTIN |
| **50** | Compound A* | ALOGLIPTIN |
| **51** | Compound A* | ALOGLIPTIN benzoate |
| **52** | Compound A* | P32/98 |
| **53** | Compound A* | P32/98 fumarate |
| **55** | Compound A* | SILDENAFIL |
| **56** | Compound A* | SILDENAFIL citrate |
| **57** | Compound A* | SILDENAFIL hemi-citrate |
| **58** | Compound A* | SILDENAFIL mesilate |
| **59** | Compound A* | VARDENAFIL |
| **60** | Compound A* | VARDENAFIL hydrochloride |
| **61** | Compound A* | VARDENAFIL dihydrochloride |
| **62** | Compound A* | TADALAFIL |
| **63** | Compound A* | UDENAFIL |
| **64** | Compound A* | AVANAFIL |
| **65** | Compound A* | AVANAFIL besilate |
| **66** | Compound A* | PRAMLINTIDE |
| **67** | Compound A* | PRAMLINTIDE acetate |
| **68** | Compound A* | cinnamon |
| **69** | Compound A* | BAY-27-9955 |
| **70** | Compound A* | INGLIFORIB |
| **71** | Compound A* | MB-06322 |
| **72** | Compound A* | MB-05032 |
| **73** | Compound A* | MB-05032 hydrobromide |
| **74** | Compound A* | AVE-1625 |
| **75** | Compound A* | RIMONABANT |
| **76** | Compound A* | RIMONABANT hydrochloride |
| **77** | Compound A* | SURINABANT |
| **78** | Compound A* | HMR-1426 |
| **79** | Compound A* | CETILISTAT |
| **80** | Compound A* | SIBUTRAMINE |
| **81** | Compound A* | SIBUTRAMINE hydrochloride |

| | | |
|---|---|---|
| Compound A* represents Compound A or a pharmaceutically acceptable salt of Compound A, in particular the hydrochloride salt of Compound A. | | |

**Table 4: Preferred triple combinations:**

| **Example Number** | **Triple Combination** | | |
|---|---|---|---|
| **82** | Compound A* | METFORMIN | Human Insulin |
| **83** | Compound A* | METFORMIN hydrochloride | Human Insulin |
| **84** | Compound A* | ROSIGLITAZONE | Human Insulin |
| **85** | Compound A* | ROSIGLITAZONE maleate | Human Insulin |
| **86** | Compound A* | ROSIGLITAZONE | METFORMIN |
| **87** | Compound A* | ROSIGLITAZONE maleate | METFORMIN |
| **88** | Compound A* | ROSIGLITAZONE maleate | METFORMIN hydrochloride |
| **89** | Compound A* | PIOGLITAZONE | Insulin |
| **90** | Compound A* | PIOGLITAZONE dihyrochloride | Insulin |
| **91** | Compound A* | PIOGLITAZONE | METFORMIN |
| **92** | Compound A* | PIOGLITAZONE | METFORMIN hydrochloride |
| **93** | Compound A* | PIOGLITAZONE dihyrochloride | METFORMIN |
| **94** | Compound A* | PIOGLITAZONE dihyrochloride | METFORMIN hydrochloride |
| **95** | Compound A* | GLIMEPIRIDE | Insulin |
| **96** | Compound A* | GLIMEPIRIDE | METFORMIN |
| **97** | Compound A* | GLIMEPIRIDE | METFORMIN hydrochloride |
| **98** | Compound A* | GLIMEPIRIDE | ROSIGLITAZONE |
| **99** | Compound A* | GLIMEPIRIDE | ROSIGLTAZONE maleate |
| **100** | Compound A* | GLIMEPIRIDE | PIOGLITAZONE |
| **101** | Compound A* | GLIMEPIRIDE | PIOGLITAZONE dihydrochloride |

| | | | |
|---|---|---|---|
| Compound A* represents Compound A or a pharmaceutically acceptable salt of Compound A, in particular the hydrochloride salt of Compound A. | | | |

### Pharmacology

### Aim of the study was to investigate the influence of a combination of RIMONABANT Hydrochloride and Compound A on HbA_{1c} and glucose tolerance.

### Animals

Female obese db/db-mice (BKS.Cg/BomTac-m +/+ *Lep^{db}),* supplied by Taconic Europe (Denmark) were used at the age of 10-11 weeks. Animals were kept under controlled conditions (22°C, 12h light / 12h dark cycle) and received standard laboratory chow and water ad libitum. For each group a number of 15 animals were used.

### Experimental Protocol

Animals were treated with Compound A [3mg/kg orally, suspended in methocel (hypromellose, 4% aequous solution)], RIMONABANT Hydrochloride [10mg/kg orally, suspended in methocel (hypromellose, 4% aequous solution), Sequoia Research Products, Pangbourne, UK] or a combination of both once daily for 4 weeks. Vehicle treated animals served as control. HbA_{1c} levels were analyzed using the Micromat II Hemoglobin A1c Test (Bio-Rad Laboratories GmbH, 80939 München, Germany). An intraperitoneal glucose tolerance test was performed in 24h fasted animals. A glucose bolus (1g/kg body weight) was administered intraperitoneally. Blood glucose was monitored for the following time points: t = -120min, 0min (before glucose administration), 30min, 60min, 90min, 120min (after glucose administration). Blood was taken by tail-tip-bleeding (5µ1) and was determined from hemolysate using an analyzing machine (Biosen S_line, EKF Diagnostic, Germany). Data are given as mean +/- SEM. The numbers of animals were 15 per group.

### Results

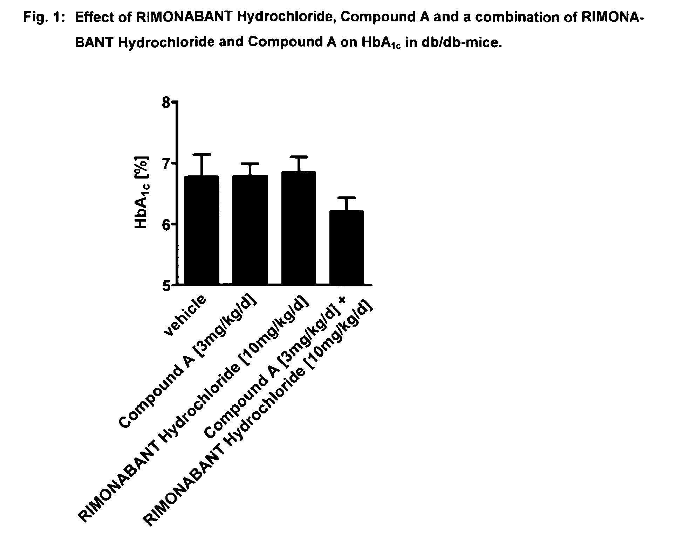

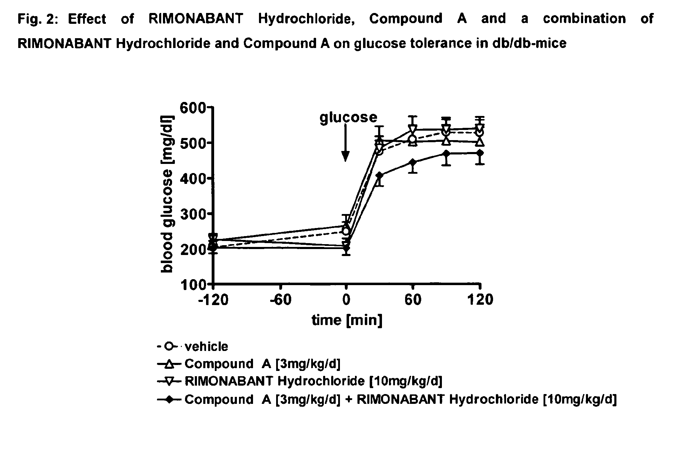

Treatment with Compound A or RIMONABANT Hydrochloride alone had no effect on HbA_{1c} (Fig. 1), the relevant diagnostic parameter for type 2 diabetes mellitus. In contrast, the combination of both compounds resulted in a decrease of HbA_{1c} (Fig. 1). Treatment with Compound A or RIMONABANT Hydrochloride alone had no effect on glucose tolerance (Fig. 2). In contrast, the combination of both compounds resulted in an improvement of glucose tolerance (Fig. 2).

### Conclusion

It is shown that a combination of RIMONABANT Hydrochloride and Compound A demonstrate over additive (synergistic) effects in the db/db mice model and therefore Compound A may be a ideal drug for combinational therapy to treat type 2 diabetes mellitus.

### Description of the Figures:

- **Fig 1:**: **Effect of RIMONABANT Hydrochloride, Compound A and a combination of RIMONA- BANT Hydrochloride and Compound A on HbA_{1c} in db/db-mice**
- **Fig 2:**: **Effect of RIMONABANT Hydrochloride, Compound A and a combination of RIMONA- BANT Hydrochloride and Compound A on glucose tolerance in db/db-mice**

## Claims

1. A pharmaceutical composition comprising a pharmaceutical formulation including Compound A or a pharmaceutically acceptable salt thereof, one other active compound or a pharmaceutically acceptable salt thereof which is used in the treatment of diabetes mellitus type 2 and/or type 1, and at least one pharmaceutically acceptable auxiliary.

2. A combination product comprising the components: (A) Compound A or a pharmaceutically acceptable salt thereof; and (B) one other active compound or a pharmaceutically acceptable salt thereof which is used in the treatment of diabetes mellitus type 2 and/or type 1, wherein each of the components (A) and (B) is formulated in admixture with at least one pharmaceutically acceptable auxiliary.

3. A kit comprising the components: (A) a pharmaceutical formulation including Compound A or a pharmaceutically acceptable salt thereof, in admixture with at least one pharmaceutically acceptable auxiliary; and (B) a pharmaceutical formulation including one other active compound or a pharmaceutically acceptable salt thereof which is used in the treatment of diabetes mellitus type 2 and/or type 1, in admixture with at least one pharmaceutically acceptable auxiliary.

4. Pharmaceutical composition, combination product or kit according to any of claims 1, 2 and 3 wherein the other active compound which is used in the treatment of diabetes mellitus type 2 and/or type 1 is a dipeptidyl-peptidase IV inhibitor or a pharmaceutically acceptable salt thereof.

5. Pharmaceutical composition, combination product or kit according to claim 4 wherein the dipeptidyl-peptidase IV inhibitor is selected from the group consisting of SITAGLIPTIN, SAXAGLIPTIN, VILDAGLIPTIN, DENAGLIPTIN, ALOGLIPTIN, P32/98 and the pharmaceutically acceptable salts of these compounds.

6. Pharmaceutical composition, combination product or kit according to claim 4 wherein the dipeptidyl-peptidase IV inhibitor is selected from the group consisting of SITAGLIPTIN, SAXAGLIPTIN, VILDAGLIPTIN, DENAGLIPTIN, P32/98 and the pharmaceutically acceptable salts of these compounds.

7. Use of Compound A or a pharmaceutically acceptable salt thereof and one other active compound or a pharmaceutically acceptable salt thereof which is used in the treatment of diabetes type 2 and/or type 1, for the manufacture of a medicament, in particular the pharmaceutical composition according to the invention, for the treatment of diabetes mellitus type 2 and/or type 1.

8. Use of Compound A or a pharmaceutically acceptable salt thereof and one other active compound or a pharmaceutically acceptable salt thereof which is used in the treatment of diabetes mellitus type 2 and/or type 1 for the manufacture of a sequentially or separately co-administrable medicament, in particular the combination product or kit according to the invention, for the treatment of diabetes type 2 and/or type 1.

9. Use according to any of claims 7 and 8 wherein the other active compound which is used in the treatment of diabetes mellitus type 2 and/or type 1 is a dipeptidyl-peptidase IV (DPPIV) inhibitor or a pharmaceutically acceptable salt thereof.

10. Use according to claim 9 wherein the dipeptidyl-peptidase IV (DPPIV) inhibitor is selected from the group consisting of SITAGLIPTIN, SAXAGLIPTIN, VILDAGLIPTIN, DENAGLIPTIN, ALOGLIPTIN, P32/98 and the pharmaceutically acceptable salts of these compounds.

11. Use according to claim 9 wherein the dipeptidyl-peptidase IV (DPPIV) inhibitor is selected from the group consisting of SITAGLIPTIN, SAXAGLIPTIN, VILDAGLIPTIN, DENAGLIPTIN, P32/98 and the pharmaceutically acceptable salts of these compounds.
